# EUROPEAN PATENT APPLICATION

(11) **EP 4 170 028 A1**
(43) Date of publication of application: **26.04.2023**
(21) Application number: 20940612.3
(22) Date of filing: 19.06.2020
(51) Int. Cl.: C12N 15/10, C40B 40/06, C40B 50/06, C12Q 1/6806, C12Q 1/6869

(54) **HIGH-COMPATIBILITY PCR-FREE LIBRARY BUILDING AND SEQUENCING METHOD**

(71) Applicant: MGI Tech Co., Ltd., Shenzhen, Guangdong 518083 (CN)
(72) Inventor: ZHAO, Xia, Shenzhen Guangdong 518083 (CN); SHEN, Hanjie, Shenzhen Guangdong 518083 (CN); LIU, Pengjuan, Shenzhen Guangdong 518083 (CN); LI, Qiaoling, Shenzhen Guangdong 518083 (CN); XI, Yang, Shenzhen Guangdong 518083 (CN); JIANG, Yuan, Shenzhen Guangdong 518083 (CN); CHEN, Fang, Shenzhen Guangdong 518083 (CN); JIANG, Hui, Shenzhen Guangdong 518083 (CN)
(74) Representative: Habermann, Hruschka & Schnabel
(86) International application number: PCT/CN2020/096987
(87) International publication number: WO 2021/253372

(57) **Abstract**

Disclosed is a PCR-free library building and sequencing method. The present invention provides a PCR-free high-throughput sequencing method, comprising the following steps: according to the size of a nucleic acid to be subjected to sequencing, performing or not performing a fragmentation treatment on the nucleic acid, so as to obtain a DNA fragment of a target size; preforming tail end repair and an A addition reaction; connecting to an adapter that includes a tag sequence; performing single-strand cyclization and rolling circle replication to form a DNA nanosphere; and performing sequencing on a machine.

## Description

### FIELD

The present disclosure relates to the technical filed of molecular biology high-throughput sequencing, and specifically, to a high-compatibility PCR-free library construction method and a high-compatibility PCR-free sequencing method, which are applicable to samples whose DNA is subjected to library construction without PCR amplification.

### BACKGROUND

Next-generation sequencing (NGS) is the most common technique used in modern molecular biology high-throughput sequencing research. NGS workflows for DNA mainly include two steps: library construction and on-machine sequencing. In the library construction step, randomly interrupted genomic fragments are amplified by standard PCR. However, for some special templates, the presence of factors such as complex secondary structures or poor thermostability may cause PCR amplification bias of the templates. Therefore, not all sequences are equally represented in the PCR-amplified library. Especially for some templates with high GC content or high AT content, it is sometimes difficult to construct a library by the PCR method. There is no obvious difference between a PCR-free library and a conventional PCR library when they are sequenced, except that PCR is not required during construction of a PCR-free library. A PCR-free library can theoretically improve data read distribution and has more uniform sequence coverage. In the on-machine sequencing step of the NGS workflows, a signal to be detected is required to be amplified by PCR in most cases. Therefore, even if a PCR-free library is constructed, problems introduced by PCR during sequencing still cannot be avoided.

At present, most library construction kits of the self-developed platforms are based on PCR construction. However, PCR library construction has the disadvantages of low coverage, GC bias, and low InDel detection accuracy and sensitivity. In addition, the PCR library construction requires a long period of time, has high requirements for fully automated instruments and laboratory, and costs a lot in library construction, labor, and depreciation. Illumina's Truseq DNA PCR-free sample preparation kit can theoretically construct a library within one day and is compatible with ever-increasing reads in the Illumina sequencing platform. However, this kit is only compatible with a library construction based on physical interruption and an input of 1 to 2 µg. Thus, this kit is mainly used for human resequencing, but it is incompatible with digestion interruption and FFPE and is also incompatible with samples of a small input such as cfDNA, thereby having a narrow application range and low flexibility. In addition, the Illumina platform adopts bridge PCR for library amplification. That is, a PCR-free constructed library is still amplified by PCR before sequencing. Therefore, the Illumina platform is not of a pure PCR-free library construction and sequencing. The available PCR-free kits have the following advantages and disadvantages as listed in Table 1.

**[Table 1] Advantages and disadvantages of existing PCR-free kits on the market**

| Product | Manufacturer | Type of sample | Interruption method | Method | Input | Total reaction time | Manual time | Price | Advantage | Disadvantage |
|---|---|---|---|---|---|---|---|---|---|---|
| TruSeq DNA PCR-free | Illumina | Genome | Physical interruption | 3-step method + Y adapter | 1/2 µg gDNA | 5h | 4h | $ 2991/9 6 RXNS | 1. Gold standard | 1. Large input |
| | | | | | | | | | | 2. Tedious steps |
| | | | | | | | | | | 3. Low conversion efficiency |
| | | | | | | | | | | 4. Incompatible with digestion interruption |
| NEXTFLEX^{®} PCR-free DNA-Seq Kit | Perkin Elmer | Genome | Digestion interruption | 3-step method + Y adapter | 500 ng to 3 µg gDNA | 4h | 1.5 h | $ 974/96 RXNS | 1. Gold standard | 1. Large input |
| | | | | | | | | | 2. Relatively small input | 2. Tedious steps |
| | | | | | | | | | | 3. Low conversion efficiency |
| Broad Institute PCR Free | Broad Institute | Genome | Physical interruption | 3-step method + Y adapter | 250 ng gDNA | NA | NA | | 1. Gold standard | 1. No kit |
| | | | | | | | | | 2. Small input | 2. Tedious steps |
| | | | | | | | | | | 3. Incompatible with digestion interruption |
| NxSeq^{®} AmpFREE Low DNA Library Kit | Lucigen | Genome, FFPE | Physical interruption | 1-tube method + Y adapter | 75 ng to 1 µg interrupted DNA | 2h 10 min | 1 h | | 1. Small input | Incompatible with cfDNA |
| | | | | | | | | | 2. Relative few steps | |
| | | | | | | | | | 3. Relative high compatibility | |
| KAPA HyperPrep Library construction Kit | KAPA Biosystems | Genome, FFPE | Digestion/phy sical interruption | 1-tube method + Y adapter | ≥ 50 ng sheared DNA (without fragment selection); ≥ 200 ng sheared DNA (without fragment selection) | 3h | 1 h | | 1. Small input | Incompatible with cfDNA |
| | | | | | | | | | 2. Relative few steps | |
| | | | | | | | | | 3. Relative high compatibility | |
| NEBNext^{®} Ultra^{™} II DNA Library Prep Kit for Illumina^{®} | NEB | Genome, FFPE | Digestion/phy sical interruption | 1-tube method + Loop adapter | 100 ng gDNA | 1.7 h | 7 min | | 1. Small input | Incompatible with cfDNA |
| | | | | | | | | | 2. Relative few steps | |
| | | | | | | | | | 3. Relative high compatibility | |
| | | | | | | | | | 4. High conversion efficiency | |
| BioDynamiPC R-free NGS DNA Library Prep Kit | BioDynami | Genome | Digestion/phy sical interruption | 1-tube method + Y adapter | 100 ng to 1 ug sheared DNA | <2 h | About 10 min | $ 1392 /48 RXNS | 1. Small input | Incompatible with cfDNA |
| | | | | | | | | | 2. High compatibility | |
| Accel-NGS^{®} 2S PCR-free DNA Library Kit | Swift Biosciences | Genome, cfDNA, FFPE | Physical/dige stion interruption | End repair + phosphorylati on + L adapter | 100 ng of high-quality sheared DNA, 10 ng cfDNA or 5 ng when pooling samples | 2 h | 1 h | | 1. Small input | Tedious steps |
| | | | | | | | | | 2. High compatibility | |
| | | | | | | | | | 3. High conversion efficiency | |
| | | | | | | | | | 4. Unique molecular identifiers (UMIs) | |

Conventional PCR library construction requires a long period of time and high costs, and cannot avoid base pairing bias introduced by PCR, thereby leading to base pairing mistake, data bias, and repetitive sequences. Furthermore, a PCR library has poor performance in Indel calling compared to a PCR-free library. The existing PCR-free kits on the market are mainly manufactured by overseas companies. High requirements for inputs are an important factor to limit application of the PCR-free kits, and it is important to reduce inputs for library construction by improving library construction efficiency. The existing PCR-free kits require the lowest input of 50 ng for gDNA and 5 ng for cfDNA. Most of the library construction kits have poor compatibility, which is reflected in the following aspects: 1) some kits are only compatible with physical interruption; 2) some kits are only compatible with normal genomic DNA and incompatible with FFPE, cfDNA, and severely degraded DNA; and 3) some kits are not equipped with a matching digestion interruption kit.

### SUMMARY

In view of the deficiencies in the prior art, the present disclosure is to provide a new quick and efficient method for constructing a PCR-free sequencing library, and the method is applicable to the sequencing platform self-developed by MGI. In the method, a library suitable for sequencing can be constructed by ligating an adapter and directly performing single-strand cyclization without PCR amplification, thereby reducing base paring mistake, data bias, and repetitive sequences that may be introduced by PCR.

In a first aspect, the present disclosure provides a PCR-free high-throughput sequencing method.

The PCR-free high-throughput sequencing method claimed in the present disclosure is the following method A, method B or method C.

The method A may include the following steps:
(A1) obtaining a DNA fragment of target size by performing or not performing fragmentation on a nucleic acid sample based on a size of the nucleic acid sample;
(A2) performing end repair and an A-tailing reaction on the product of step (A1);
(A3) ligating an adapter to the product of step (A2);
(A4) obtaining DNA nanoballs by performing single-strand cyclization on the product of step (A3) and rolling circle replication; and
(A5) loading and sequencing.

The method B may include the following steps:
(B1) obtaining a DNA fragment of target size by performing fragmentation on a nucleic acid sample based on a size of the nucleic acid sample, and performing end repair and an A-tailing reaction;
(B2) ligating an adapter to the product of step (B1);
(B3) obtaining DNA nanoballs by performing single-strand cyclization on the product of step (B2) and rolling circle replication; and
(B4) loading and sequencing.

The method C may include the following steps:
(C1) performing fragmentation on a nucleic acid sample, based on a size of the nucleic acid sample, and performing end repair at the same time to obtain a DNA fragment of target size;
(C2) performing an A-tailing reaction on the product of step (C1);
(C3) ligating an adapter to the product of step (C2);
(C4) obtaining DNA nanoballs by performing single-strand cyclization on the product of step (C3) and rolling circle replication; and
(C5) loading and sequencing.

In step (B1), or (C1), the fragmentation is performed by digesting the nucleic acid sample with fragmentmase.

Further, the fragmentmase may be, for example, NEBNext^{®} Ultra^{™} II FS DNA Module, Qiagen5X WGS Fragmentation Mix, or a self-developed enzyme for interruption.

In the method A, the method B, and the method C, the adapter contains a barcode. Preferably, the adapter includes two barcodes. The adapter including two barcodes is ligated to both ends of the nucleic acid sample to form a library having dual barcodes.

Further, the adapter is formed by annealing two partially complementary single-stranded nucleic acids, and the two barcodes are located in a non-complementary region of the two single-stranded nucleic acids.

In step (A1), (B1), or (C1), the nucleic acid sample may be DNA or RNA.

Further, the DNA is genomic DNA, a naturally occurring small-molecule DNA, or an amplified DNA product.

The naturally occurring small-molecule DNA may be, for example, cfDNA. The amplified DNA product may be, for example, an MDA product, a cDNA product, or an amplicon.

A starting sample directly used in the PCR-free library construction method of the present disclosure may be a non-DNA sample such as a blood or saliva sample.

In step (A1), the fragmentation may be performed through physical interruption or digestion interruption.

The physical interruption may be, for example, ultrasonic interruption.

If the nucleic acid sample is larger than the DNA fragment of target size (e.g., genomic DNA), the nucleic acid sample is required to be interrupted, and the DNA fragment of target size is selected by a magnetic bead method. If the nucleic acid sample is not larger than the DNA fragment of target size (e.g., cfDNA, which is a small DNA fragment and has a concentrated main band), the nucleic acid sample is not required to be subjected to fragmentation.

If the nucleic acid sample is RNA, the RNA is subjected to reverse transcription to obtain DNA; and the fragmentation is performed on the RNA or the DNA obtained by the reverse transcription of the RNA.

In step (A1), (B 1), or (C1), a size of the DNA fragment of target size may ranges from 150 bp to 800 bp, for example, 300 bp to 500 bp.

In step (A2), the end repair and the A-tailing reaction are performed in one step by mixing and reacting an end repair-A-tailing reaction solution with the product of step (A1), to obtain the product of step (A2).

The end repair-A-tailing reaction solution contains a T4 polynucleotide kinase buffer (T4 PNK buffer), adenylate deoxyribonucleic acids (dATP), a mixed deoxyribonucleic acid solution (dNTPs), T4 DNA polymerases, T4 polynucleotide kinases (T4 PNK), and Taq DNA polymerases (rTaq).

Further, in the end repair-A-tailing reaction solution, the adenylate deoxyribonucleic acids (dATP), the mixed deoxyribonucleic acid solution (dNTPs), the T4 DNA polymerases, the T4 PNK, the Taq DNA polymerases (rTaq) satisfy a ratio of 50 nmol: 12.5 nmol (for each dNTP): 6 U: 10 U: (2 to 5) U.

In a specific example of the present disclosure, 10× T4 PNK buffer, an adenylate deoxyribonucleic acid solution (dATP) at a concentration of 100 mM, a mixed solution of 4 kinds of deoxyribonucleic acid (dNTPs) in which the concentration of each deoxyribonucleic acid is 25 mM, T4 DNA polymerases at a concentration of 3 U/µL, T4 PNK at a concentration of 10 U/µL, and Taq DNA polymerases (rTaq) at a concentration of 5 U/µL are mixed in a volume ratio of 5: 0.5: 0.5: 2: 1: (0.4-1), to prepare the end repair-A-tailing reaction solution.

In step (A2), the end repair-A-tailing reaction solution is mixed with the product of step (A1) in volume ratio of 1: 4.

In step (A2), the end repair-A-tailing reaction solution and the product of step (A1), after being mixed, react under the following conditions: 1) at 14°C for 15 min, at 37°C for 25 min, and at 65°C for 15 min; and kept at 4°C, the heated lid of the PCR instrument is set to 70°C; or, 2) incubated at 37°C for 10 min and at 72°C for 15 min, and cooled to 4°C at a rate of 0.1 s.

In step (B1), the fragmentation, the end repair, and the A-tailing reaction are performed in one step by mixing and reacting a fragmentation-end repair-A-tailing reaction solution with the nucleic acid sample, to obtain the product of step (B1).

The fragmentation-end repair-A-tailing reaction solution contains fragmentmase, a fragmentmase reaction buffer, adenylate deoxyribonucleic acids, a mixed deoxyribonucleic acid solution, T4 DNA polymerases, Taq DNA polymerases, and a TE buffer.

Further, in the fragmentation-end repair-A-tailing reaction solution, the adenylate deoxyribonucleic acids, the mixed deoxyribonucleic acid solution, the T4 DNA polymerases, and the Taq DNA polymerases may satisfy a ratio of 170 nmol: 57.5 nmol: 3 U: 5 U. The amount of enzyme for interruption is determined according to the instruction or results of multiple experiments.

Further, in step (B1), the fragmentation-end repair-A-tailing reaction solution and the nucleic acid sample, after being mixed, may be incubated at 37°C for 10 to 20 min and at 65°C for 30 min; and the temperature of the mixture is cooled to 4°C. The heated lid of the PCR instrument is set to 70°C.

In step (C1), the fragmentation and the end repair are performed in one step by mixing and reacting a fragmentation-end repair reaction solution with the nucleic acid sample, to obtain the product of step (C1).

The f fragmentation-end repair reaction solution contains fragmentmase, a fragmentmase reaction buffer, a mixed deoxyribonucleic acid solution, DNA polymerase I, and MgCl₂, and enzyme-free water.

Further, in the fragmentation-end repair reaction solution, the mixed deoxyribonucleic acid solution, the DNA polymerases I, and the MgCl₂ may be satisfy a ratio of 75 nmol (for each dNTP): 20 U: 0.3 µmol. The amount of enzyme for interruption is determined according to the instruction or results of multiple experiments.

Further, in step (C1), the fragmentation-end repair reaction solution and the nucleic acid sample, after being mixed, may react at 37°C for 30 min, and the temperature of the mixture is kept at 4°C. The heated lid of the PCR instrument is set to 70°C. After the reaction is completed, the sample is collected and placed on ice immediately, and TE is added to make up the total volume of the sample to 30 µL.

In step (C2), an A-tailing reaction solution for the A-tailing reaction performed on the product of (C1) contains a T4 PNK buffer, adenylate deoxyribonucleic acids (dATP), a mixed deoxyribonucleic acid solution (dNTPs), and Taq DNA polymerases (rTaq).

Further, in the A-tailing reaction solution, the adenylate deoxyribonucleic acids (dATP), the mixed deoxyribonucleic acid solution (dNTPs), and the Taq DNA polymerases (rTaq) satisfy a ratio of 50 nmol: 8.75 nmol (for each dNTP): 1 U.

In a specific example of the present disclosure, 10× T4 PNK buffer, an adenylate deoxyribonucleic acid solution (dATP) at a concentration of 100 mM, a mixed solution of 4 kinds of deoxyribonucleic acid in which the concentration of each deoxyribonucleic acid is 25 mM, Taq DNA polymerases (rTaq) at a concentration of 5 U/µL, and enzyme-free water are mixed in volume ratio of 5: 0.5: 0.35: 0.2: 1, to prepare the A-tailing reaction solution.

In step (A3), (B2), or (C3), the adapter is formed by annealing a B strand and a T strand. A 3'-end of the B strand is complementary with a 5'-end of the T strand, and the remaining region of the B strand is non-complementary and unpaired with the remaining region of the T stand. The 3'-end of the B strand has a protruding dT. The non-complementary region of the B strand and/or the non-complementary region of the T strand contain a barcode for identifying different samples. A 5'-end of the B strand and the 5'-end of the T strand are each modified with a phosphate group or ligated with a single-stranded oligonucleotide fragment having a U-base at 3'-end.

If the adapter contains a single-stranded oligonucleotide fragment having a U-base at 3'-end, the adapter is required to be subj ected to USER enzyme treatment. The USER enzyme treatment and the ligation may be performed simultaneously, or the USER enzyme treatment may be performed after the ligation of the adapter and purification.

Further, the adapter may be any one of the adapter 1, adapter 2, adapter 3, and adapter 4.

The adapter 1 is formed by annealing a single-stranded DNA set forth in SEQ ID NO: 1 with a phosphate group-modified 5'-end and a single-stranded DNA set forth in SEQ ID NO: 2 with a phosphate group-modified 5'-end.

### Phosphorylated adapter B strand:

/Phos/GAACGACATGGCTACGATCCGACTT (SEQ ID NO: 1); and
phosphorylated adapter T strand:

/Phos/AGTCGGAGGCCAAGCGGTCTTAGGAAGACAANNNNNNNNNNCAACTCCTT
GGCTCACA (SEQ ID NO: 2).

The adapter 1 is a Y adapter, and after the adapter is ligated, the product can be directly subjected to cyclization or simultaneously subjected to cyclization and rolling circle replication.

The adapter 2 is formed by annealing a single-stranded DNA set forth in SEQ ID NO: 3 or SEQ ID NO: 4 and a single-stranded DNA set forth in SEQ ID NO: 2.

### Adapter B strand having a U base (design 1):

TTGTCTTCCUGAACGACATGGCTACGATCCGACTT (SEQ ID NO: 3);
adapter B strand having a U base (design 2):
   TTGTCTTCCTAAGUGAACGACATGGCTACGATCCGACTT (SEQ ID NO: 4); and
phosphorylated adapter T strand:

/Phos/AGTCGGAGGCCAAGCGGTCTTAGGAAGACAANNNNNNNNNNCAACTCCTT
GGCTCACA (SEQ ID NO: 2).

The adapter 2 is a Bubble U-shaped adapter, and can be simultaneously subjected to ligation and USER enzyme treatment. The product can be directly and simultaneously subjected to cyclization or subjected to cyclization and rolling circle replication.

The adapter 3 is formed by annealing a single-stranded DNA set forth in SEQ ID NO: 5 and a single-stranded DNA set forth in SEQ ID NO: 6.

### Adapter B strand:

TTGTCTTCCUTCTCAGTACGTCAGCAGTTNNNNNNNNNNCAACTCCTTGGCTCA
CAGAACGACATGGCTACGATCCGACTT (SEQ ID NO: 5); and

phosphorylated adapter T strand:

/Phos/AGTCGGAGGCCAAGCGGTCTTAGGAAGACAANNNNNNNNNNCTGATAAGG
TCGCCATGCC (SEQ ID NO: 6).

The adapter 3 is a dual-barcode U-shaped adapter, and can be simultaneously subjected to ligation and USER enzyme treatment. The product can be directly and simultaneously subjected to cyclization or subjected to cyclization and rolling circle replication.

The adapter 4 is formed by annealing a single-stranded DNA set forth in SEQ ID NO: 7 and a single-stranded DNA set forth in SEQ ID NO: 6.

### Phosphorylated adapter B strand:

/Phos/TCTCAGTACGTCAGCAGTTNNNNNNNNNNCAACTCCTTGGCTCACAGAACG
ACATGGCTACGATCCGACTT (SEQ ID NO: 7); and

phosphorylated adapter T strand:

/Phos/AGTCGGAGGCCAAGCGGTCTTAGGAAGACAANNNNNNNNNNCTGATAAGG
TCGCCATGCC (SEQ ID NO: 6).

The adapter 4 is a dual-barcode Y adapter sequence, and after the adapter is ligated, the product can be directly and simultaneously subjected to cyclization or subjected to cyclization and rolling circle replication.

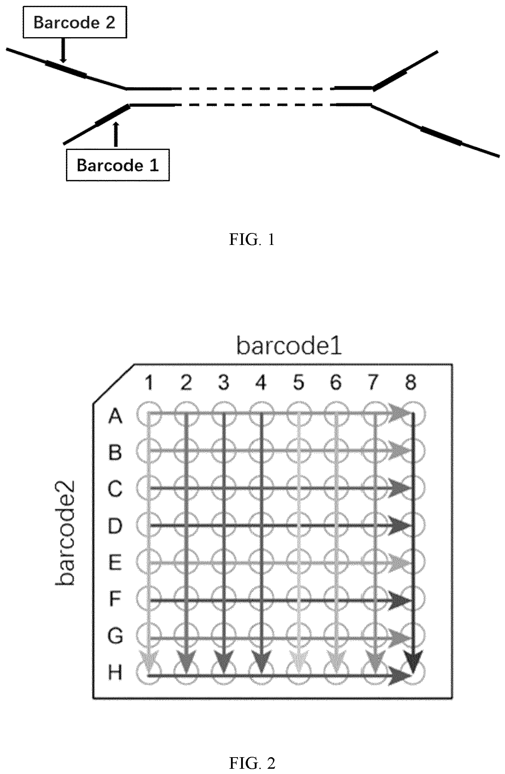

A Y portion of the two strands of the adapter have each a barcode, which is added to a library by ligation (see FIG. 1). After PCR, the library has barcodes at both ends, having the advantages that the samples can be mixed immediately after the ligation of adapters, and a PCR-free library can be used universally.

After barcodes are ligated to both ends of a library, types of barcodes are greatly increased through the combinations of the barcodes at both ends, thereby achieving sequencing for many mixed libraries. As shown in FIG. 2, barcodes in the horizontal and vertical columns are added to both ends of a library, respectively, and types of barcodes are increased through the combinations of the barcodes at both ends. The barcodes at both ends of a library may be the same or different. In the example shown in FIG. 2, by only designing 8 types of barcodes, 64 combinations can be obtained through the combinations of the barcodes at both ends, solving the problem that a variety of barcodes is necessarily designed for a library having a single barcode to achieve sequencing for a mixture of corresponding multiple libraries.

In addition, in the case that each barcode is used uniquely at each of the both ends, barcode skipping errors during library construction or sequencing can be greatly eliminated by use of the unique correspondence between the barcodes at both ends.

In each adapter sequence, the 5'-end is on the left, the 3'-end is on the left, "//" represents a modifying group, and "phos" represents phosphorylation. In the T strand of adapter, a sequence of 10 bases, i.e., NNNNNNNNNN, represents a barcode, and N may be A, T, C or G. The barcode is used to identify different samples. Samples having different barcodes can be used to construct a mixed library.

The adapter sequence is not limited to the above sequences. Even if the sequence is modified, a similar effect can be achieved, as long as the structure can be sequenced on BGI/MGI platform.

In addition, by modifying the adapter sequence, for example, by adding a corresponding barcode NNN (N may be A, T, C or G, and in specific examples, the length of N can be set according to experimental objectives) to the adapter sequence, unique molecular identifiers (UMIs) can also be added while ligating the adapter to the library. The UMI can be used as an identifier for each sample strand and mostly used for detection of low-frequency variants. The UMI is usually adjacent to a target nucleic acid or a barcode, and shares one sequencing primer with the target nucleic acid or the barcode during sequencing to achieve continuous sequencing. The UMI may be separated from the target nucleic acid and the barcode, and uses a self-developed sequencing primer during sequencing.

In step (A3), (B2), or (C3), the adapter is ligated to the product of step (A2), (B 1), or (C2) by mixing and reacting the adapter and the product of step (A2), (B1), or (C2) with a ligation reaction solution, to obtain the product of step (A3), (B2), or (C3).

The ligation reaction solution contains a T4 PNK buffer, adenylate ribonucleic acids (ATP), PEG8000, T4 DNA ligases, and enzyme-free water.

Further, in the ligation reaction solution, the adenylate ribonucleic acids (ATP), the PEG8000, and the T4 DNA ligase satisfy a ratio of 0.8 µmol of adenylate ribonucleic acids: (10 to 16) µL of 50% PEG8000 (e.g., Rigaku's products): (1,200 to 3,000) U of T4 DNA ligases, for example, 0.8 µmol of adenylate ribonucleic acids: 16 µL of 50% PEG8000: 3,000 U of T4 DNA ligase.

In a specific example of the present disclosure, 10× T4 PNK buffer, adenylate ribonucleic acids (ATP) at a concentration of 0.1 M, PEG8000 at a concentration of 50% (e.g., Rigaku's products), T4 DNA ligases at a concentration of 600 U/µL, and enzyme-free water are mixed in volume ratio of 3: 0.8: 16: 5: 0.2, to prepare the ligation reaction solution.

In step (A3), (B2), or (C3), the adapter, the product of step (A2), (B1), or (C2), and the ligation reaction solution are mixed by mixing an adapter solution containing the adapter and the product of step (A2), (B1), or (C2) with the ligation reaction solution in a volume ratio of (1 to 5): 50: (25 to 29); specifically, e.g. 5: 50: 25 or 1: 50: 29. A concentration of the adapter in the adapter solution is 6 µM or 1 µM.

In step (A3), (B2), or (C3), the adapter, the product of step (A2), (B1), or (C2), and the ligation reaction solution, after being mixed, may react at 25°C for 10 to 30 min (e.g., 30 min); and the temperature of the mixture is kept at 4°C. The heated lid of the PCR instrument is set to 30°C.

In the present disclosure, the adapters compatible with DNBSEQ^{™} series sequencing platform and multiple high-throughput sequencing platforms self-developed by MGI, such as single-barcode adapters and dual-barcode adapters, are designed. The adapter sequence contains the barcode sequences, which can be used to identify multiple samples at the same time, and the samples with different barcode sequences can be used to construct a mixed library. The optimized adapter ligation system used in the present disclosure can still ensure high ligation efficiency despite a small input of adapters (e.g., a molar ratio of adapters to DNAs is 5: 1 to 50: 1), which advantageously avoiding adapter contamination caused by too many adapters and improving the efficiency of converting DNA templates into sequencing libraries with adapters at both ends.

In step (A4), (B3), or (C4), the product of step (A3), (B2), or (C3) is purified before the single-strand cyclization and the rolling circle replication.

Further, the purification is a magnetic bead purification (e.g., using XP magnetic beads or various indigenous magnetic beads for purification).

A TE buffer is added to the product of step (A3), (B2), or (C3), XP magnetic beads (Beckman Coulter) or various indigenous magnetic beads for purification are added to purify the product, and a collected product is dissolved in the TE buffer.

In step (A4), (B3), or (C4), specifically, said obtaining DNA nanoballs by performing the single-strand cyclization on the product of step (A3), (B2), or (C3) and the rolling circle replication includes any one of:
(a1) sequentially performing single-strand cyclization, linear single strand digestion, purification, and rolling circle replication, to obtain the DNA nanoballs; and
(a2) performing the single-strand cyclization and the rolling circle replication in one step, to obtain the DNA nanoballs.

In step (a1), the single-strand cyclization may be performed by a manner I or manner II.

The manner I includes the following steps:
I-1: incubating the product of step (A3), (B2), or (C3) at 95°C for 3 min and at 4°C for 10 min to obtain an incubation product; and
I-2: mixing and reacting a single-strand cyclization reaction solution 1 with the incubation product of step I-1 to obtain a cyclization product

The single-strand cyclization reaction solution 1 contains a TA buffer, adenylate ribonucleic acids (ATP), mediation fragments, T4 DNA ligases, and enzyme-free water. The mediation fragments are each a single-stranded DNA, which has a 5'-end reversely complementary to a'-end of the B strand constituting the adapter, and a 3'-end reversely complementary to a 3'-end of the T strand constituting the adapter.

In the present disclosure, if the adapter is the adapter 1 or the adapter 2 described above, the mediation fragments have a nucleotide sequence set forth in SEQ ID NO: 8.

Further, in the single-strand cyclization reaction solution 1, the adenylate ribonucleic acids (ATP), the mediation fragments, and the T4 DNA ligase satisfy a ratio of 60 nmol: 62.5 pmol: 600 U.

In a specific example of the present disclosure, 10× TA buffer, adenylate ribonucleic acids (ATP) at a concentration of 100 mM, mediation fragments at a concentration of 25 µM, T4 DNA ligases at a concentration of 600 U/µL, and enzyme-free water are mixed in volume ratio of 6: 0.6: 2.5: 1: 1.9, to prepare the single-strand cyclization reaction solution 1.

The single-strand cyclization reaction solution 1 is mixed with the incubation product in volume ratio of 48: 12.

The single-strand cyclization reaction solution 1 and the incubation product, after being mixed, are incubated at 37°C for 60 min; and the temperature of the mixture is kept at 4°C. The heated lid of the PCR instrument is set to 42°C.

The manner II includes the following steps: mixing the product of step (A3), (B2), or (C3) with the mediation fragments and a NaOH solution; incubating the mixture at the room temperature for 5 min; mixing the mixture with a Tris-HCL solution; and adding a single-strand cyclization reaction solution 2 for reaction to obtain a cyclization product.

The concentration of the NaOH solution is 2 M, and the concentration of the Tris-HCL solution is 1 M, and the pH is 6.8.

Further, the NaOH, the mediation fragments, and the Tris-HCL satisfy 5 µmol: 100 pmol: 5 µmol.

The single-strand cyclization reaction solution 2 contains a TA buffer, adenylate ribonucleic acids (ATP), and T4 DNA ligases.

In the present disclosure, in the single-strand cyclization reaction solution 2, a ratio of the adenylate ribonucleic acids (ATP) to the T4 DNA ligases is 60 nmol: 240 U.

In a specific example of the present disclosure, 10× TA buffer, adenylate ribonucleic acid (ATP) at a concentration of 100 mM, and T4 DNA ligases at a concentration of 600 U/µL are mixed in volume ratio of 6: 0.6: 0.4, to prepare the single-strand cyclization reaction solution 2.

In a specific example of the present disclosure, the product of step (A3), (B2), or (C3), the mediation fragments at a concentration of 20 µM, the NaOH solution at a concentration of 2 M, and the 1 M Tris-HCL to the single-strand cyclization reaction solution 2 satisfy a volume ratio of 48: 5: 2.5: 5: 7.

In the manner II, the reaction may occur at 37°C for 30 min; and the temperature of the mixture is kept at 4°C. The heated lid of the PCR instrument is set to 42°C.

In step (a1), the linear single strand digestion includes the following steps:
step 3: mixing and reacting a digestion reaction solution with the cyclization product to obtain a digestion product.

The digestion reaction solution contains a TA buffer, ExoI enzymes, ExoIII enzymes, and enzyme-free water.

Further, in the digestion reaction solution, a ratio of the ExoI enzymes to the ExoIII enzymes may be 4 U: 1 U.

In a specific example of the present disclosure, 10× TA buffer, ExoI enzymes at a concentration of 20 U/µL, ExoIII enzymes at a concentration of 10 U/µL, and enzyme-free water are mixed in a volume ratio of 0.4: 2: 1: 0.6, to prepare the digestion reaction solution.

The digestion reaction solution is mixed with the cyclization product in volume ratio of 4: 60 or 4: 67.5.

The digestion reaction solution and the cyclization product, after being mixed, may be incubated at 37°C for 30 min.

At this step, EDTA may be added to and uniformly mixed with the reaction product.

In step (a1), the purification is a magnetic bead purification. Specifically, XP magnetic beads are used to purify and collect the product of the previous step, and the product is dissolved in a TE buffer.

In step (A5), (B4), or (C5), the sequencing may be specifically performed on BGISEQ, MGISEQ or DNBSEQ^{™} series sequencing platform.

In a second aspect, the present disclosure provides a method for constructing a DNA library applicable to PCR-free high-throughput sequencing.

The method for constructing a DNA library applicable to PCR-free high-throughput sequencing, as provided in the present disclosure, may include steps (A1) to (A4), (B1) to (B3), or (C1) to (C4) as described in the first aspect.

In a third aspect, the present disclosure provides a DNA library constructed by the method described in the second aspect.

In a fourth aspect, the present disclosure provides an adapter.

The adapter claimed in the present disclosure is the adapter described in the first aspect.

In a fifth aspect, the present disclosure provides a kit.

The kit provided in the present disclosure contains the adapter described in the fourth aspect and all or some of:
(b 1) the end repair-A-tailing reaction solution described in the first aspect;
(b2) the fragmentation-end repair-A-tailing reaction solution described in the first aspect;
(b3) the fragmentation-end repair reaction solution described in the first aspect;
(b4) the ligation reaction solution described in the first aspect;
(b5) the single-strand cyclization reaction solution 1 or the single-strand cyclization reaction solution 2 described in the first aspect; and
(b6) the digestion reaction solution described in the first aspect.

In a sixth aspect, the present disclosure provides a system.

The system provided in the present disclosure contains the kit described in the fifth aspect, and a DNBSEQ^{™} sequencing reagent and/or device.

In a seventh aspect, the present disclosure provides use of the DNA library described in the third aspect, the adapter described in the fourth aspect, the kit described in the fifth aspect or the system described in the sixth aspect in PCR-free high-throughput sequencing.

In an eighth aspect, the present disclosure provides use of the adapter described in the fourth aspect or the kit described in the fifth aspect in construction of the DNA library described in the third aspect.

### BRIEF DESCRIPTION OF DRAWINGS

FIG. 1 is a schematic diagram illustrating a combination of barcodes at both ends of a library when a dual-barcode adapter is used.
FIG. 2 is a schematic diagram illustrating a ligation reaction between dual-barcode adapters and nucleic acids to be sequenced.
FIG. 3 shows results of two interrupted human whole genome gDNA samples in Example 1, where lane 1 and lane 2 represent two parallel digestion interruption products of 1 µg of NA12878 gDNA.
FIG. 4 shows results of a 6% TBU gel detection of a single-stranded circular library in Example 1, where lane 1 and lane 2 represent two parallel digestion products of ssCircular DNA.
FIG. 5 shows PCR results of a ligation product in Example 2, where lane 1 and lane 2 represent two parallel PCR products of the ligation product.
FIG. 6 shows analysis results by taking 5 MB and 30 MB of sequencing data in Example 2, where diagram **a** illustrates effective alignment rates; diagram **b** illustrates repetition rates; and diagram **c** illustrates GC contents.

### DESCRIPTION OF EMBODIMENTS

The following examples are only for a better understanding of, rather than limiting, the present disclosure. Unless otherwise indicated, experimental methods in the following examples are conventional methods. Unless otherwise indicated, test materials used in the following examples are purchased from conventional biochemical reagent stores. Quantitative tests in the following examples are all repeated three times and test results are averaged.

**Example 1** Construction of a human whole genome library with a self-developed platform PCR-free library construction kit (based on digestion interruption), and sequencing thereof

Experimental objective: constructing a whole genome library from a human gDNA sample by using an MGI PCR-free kit in combination with an NEB digestion interruption kit.

Sources of experimental samples: NA12878 standard DNA (catalog number: NA12878, manufacturer: CORIELL INSTITUTE).

### 1. Interruption of DNA sample with the NEB digestion interruption kit

1 µg of standard DNA (dissolved in TE) was placed into each tube and subjected to digestion interruption with NEBNext^{®} Ultra^{™} II FS DNA Module (NEB), and the volume of an interruption system was 35 µL. NEBNext Ultra II FS Reaction Buffer was thawed in advance and vortex-mixed, NEBNext Ultra II FS Enzyme Mix was uniformly mixed in an upside-down manner and placed on ice. A reaction system shown in Table 1 was prepared on ice.

**[Table 1] NEB digestion interruption reaction system for the DNA sample**

| Component | Amount |
|---|---|
| NEBNext Ultra II FS Reaction Buffer | 7 µL |
| gDNA (dissolved in TE) | X µL |
| TE | 26-X µL |
| Total volume | 33 µL |

After NEBNext Ultra II FS Enzyme Mix was uniformly pipetted, 2 µL of NEBNext Ultra II FS Enzyme Mix was added to the sample, the mixture was gently and uniformly pipetted 6-8 times (vortex-mixing was prohibited), subjected to transient centrifugation, and immediately placed in a thermocycler for reaction, the reaction conditions were as follows: 4°C forever; 37°C for 10 min; 65°C for 30 min; and 4°C forever; the heated lid of the PCR instrument was set to 70°C. After the reaction was completed, the sample was collected and placed on ice, and TE was added to make up the volume of the sample to 65 µL.

### 2. Selection of DNA fragment

(1) 100 µL of interrupted sample was taken and transferred into a new 1.5 mL non-stick tube, 60 µL of XP magnetic beads was added to and uniformly mixed with the sample by shaking, and allowed to bind to DNA at the room temperature for 10 min. The tube was placed onto a magnetic rack, the magnetic beads were allowed to bind to DNA for 2 min (until the liquid became clear), and the supernate was carefully taken by suction and transferred into a new 1.5 mL EP tube (the supernate was reserved at this step). 15 µL of XP magnetic beads was added to and uniformly mixed with the supernate by shaking, and allowed to bind to DNA at the room temperature for 10 min, the tube was placed onto the magnetic rack, the magnetic beads were allowed to bind to DNA for 2 min (until the liquid became clear), and the supernate was removed by suction.
(2) 500 µL of 75% ethanol was placed into the non-stick tube on the magnetic rack, the tube cap was closed, the mixture in the tube was uniformly mixed, and the supernate was removed. After washing with 500 µL of 75% ethanol again, residual ethanol was removed as much as possible by using a pipette with a small measurement range, and the magnetic beads were air-dried at the room temperature.
(3) The magnetic beads were resuspended in and uniformly mixed with 42 µL of TE by shaking, and allowed to bind to DNA at the room temperature for 10 min; the tube was placed onto the magnetic rack; the magnetic beads were allowed to bind to DNA for 2 min (until the liquid became clear); and 40 µL of supernate was carefully taken by suction and transferred into a new 1.5 mL EP tube for next reaction, or it stored in a refrigerator at -20°C.

### 3. Quantitation and normalization of the sample

2 µL of purified DNA was taken and subjected to Qubit dsDNA HS quantitation. The selected DNA fragment was normalized according to the concentration determined by Qubit quantitation. The mass of the DNA fragment was adjusted to 150 ng, and 1× TE was added to make up the total volume of 40 µL. If necessary, the normalized samples can be stored in a refrigerator at -20°C.

The size of the obtained DNA fragment was 300 bp to 500 bp.

### 4. End repair and A-tailing

First, an end repair-A-tailing reaction solution was prepared according to Table 2.

**[Table 2] Composition of an end repair-A-tailing reaction solution**

| Component | Amount |
|---|---|
| T4 10× PNK buffer (Enzymatics) | 5 µL |
| dATP (100 mM) (Enzymatics) | 0.5 µL |
| dNTPs (each 25 mM) (Enzymatics) | 0.5 µL |
| T4 DNA polymerase (3 U/µL) (Enzymatics) | 2 µL |
| T4 PNK (10 U/µL) (Enzymatics) | 1 µL |
| rTaq (5 U/µL) (Enzymatics) | 1 µL |
| Total volume | 10 µL |

10 µL of prepared end repair-A-tailing reaction solution was added to and uniformly vortex-mixed with 40 µL of product of step 3, the mixture was subjected to transient centrifugation, and the total volume of the mixture was made up to 50 µL. The reaction sample was placed into the PCR instrument for reaction, the reaction conditions were as follows: 14°C for 15 min; 37°C for 25 min; 65°C for 15 min; and 4°C forever, and the heated lid of the PCR instrument was set to 70°C.

### 5. Ligation of an adapter

An adapter sequence used in the present protocol is as follows (in the present example, the 5'-end of the sequence is on the left side, the 3'-end of the sequence is on the right side, "//" represents a modifying group, "phos" represents phosphorylation, and the underlined bases represent a barcode of 10 bases).

### Phosphorylated adapter B strand:

/Phos/GAACGACATGGCTACGATCCGACTT (SEQ ID NO: 1); and
phosphorylated adapter T strand: 
/Phos/AGTCGGAGGCCAAGCGGTCTTAGGAAGACAANNNNNNNNNNCAACTCCTT
GGCTCACA (SEQ ID NO: 2),
 where N may be A, T, C or G.

Preparation of adapter: 20 µL of adapter B strands (100 µM), 20 µL of adapter T strands (100 µM), and 40 µL of 2× adapter buffer (components: 50 mM Tris-HCl (pH=8.0), 0.1 mM EDTA, and 50 mM NaCl) were mixed to prepare adapters A (25 µM). The adapters A were placed at the room temperature for more than half an hour and then diluted to a use concentration or stored at -20°C. Before use, the adapters A (25 µM) were diluted with TE prepare adapters B (6 µM).

5 µL of prepared adapters B (6 µM) was added to and thoroughly mixed with the product of step 4.

A ligation reaction solution was prepared according to Table 3.

**[Table 3] Composition of a ligation reaction solution**

| Component | Amount |
|---|---|
| 10× T4 PNK buffer (Enzymatics) | 3 µL |
| 0.1 M ATP (Thermo) | 0.8 µL |
| 50% PEG8000 (Rigaku) | 16 µL |
| T4 DNA ligase (600 U/µL) (Enzymatics) | 5 µL |
| Enzyme-free water (Sigma) | 0.2 µL |
| Total volume | 25 µL |

The prepared ligation reaction solution was uniformly vortex-mixed with the mixture of the adapters B and the product of step 4, and the mixture was subjected to transient centrifugation. The reaction sample was placed into the PCR instrument for reaction; the reaction conditions were as follows: 25°C for 30 min; and 4°C hold; and the heated lid of the PCR instrument was set to 30°C. After the reaction was completed, 20 µL of TE buffer was added, 50 µL of XP magnetic beads was added for purification, and the collected product was dissolved in 50 µL of TE buffer.

### 6. Single-strand cyclization

48 µL of purified product was incubated at 95°C for 3 min and at 4°C for 10 min.

A single-strand cyclization reaction solution was prepared according to Table 4.

**[Table 4] Composition of a single-strand cyclization reaction solution**

| Component | Amount |
|---|---|
| 10× TA buffer (Epicentre) | 6 µL |
| 100 mM ATP (Thermo) | 0.6 µL |
| 20 µM mediation fragments | 2.5 µL |
| T4 DNA ligase (600 U/µL) (Enzymatics) | 1 µL |
| Enzyme-free water (Sigma) | 1.9 µL |
| Total volume | 12 µL |

12 µL of the prepared single-strand cyclization reaction solution was uniformly vortex-mixed with 48 µL of thermal denaturation product, and the mixture was subjected to transient centrifugation. The reaction sample was placed into the PCR instrument for reaction, the reaction conditions were as follows: 37°C for 60 min; and 4°C hold, and the heated lid of the PCR instrument was set to 42°C.

20 µM fragments for mediation have a corresponding complementary sequence to be ligated to both ends of the single strand. The corresponding complementary sequence is (in the present example, the 5'-end of the sequence is on the left side, and the 3'-end of the sequence is on the right side): GCCATGTCGTTCTGTGAGCCAAGG (SEQ ID NO: 8).

### 7. Digestion of a linear single strand

A digestion reaction solution was prepared according to Table 5.

**[Table 5] Composition of a digestion reaction solution**

| Component | Amount |
|---|---|
| 10× TA buffer (Epicentre) | 0.4 µL |
| ExoI (20 U/µL) (Enzymatics) | 2 µL |
| ExoIII (10 U/µL) (Enzymatics) | 1 µL |
| Enzyme-free water | 0.6 µL |
| Total volume | 4 µL |

4 µL of prepared digestion reaction solution was added to and uniformly mixed with 60 µL of reaction product of the previous step. The mixture was incubated at 37°C for 30 min, and added and uniformly mixed with 3 µL of EDTA (500 mM, Ambion). A product was purified and collected with 120 µL of XP magnetic beads, and dissolved in 30 µL of TE buffer.

### 8. Quantitation of a single-stranded circle

The single-stranded cyclization product obtained through the digestion of the linear single strand in the previous step was quantitated by using a Qubit ssDNA Assay Kit.

### 9. Sequencing

DNA nanoballs were prepared with the constructed single-stranded circular DNA library and sequenced on MGISEQ-2000 PE150. The sequencing and data analysis followed the standard operating process of MGISEQ-2000 PE150.

### 10. Library construction and sequencing results of the present example

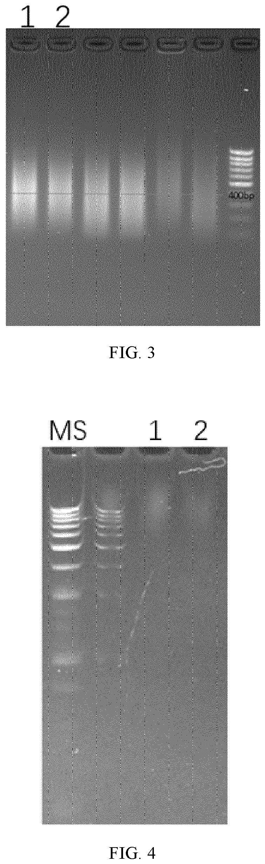

FIG. 3 shows interruption results of two human whole genome gDNA samples (parallel repetitive library construction results of NA12878 standard DNA). FIG. 4 shows a 6% TBU gel detection result of a single-stranded circular library. It can be seen from FIG. 3 and FIG. 4 that the digestion interruption and library construction results are normal, indicating that the library construction system is compatible with digestion interruption.

Table 6 illustrates the sequencing quality of the human sample WGS PCR-free library (based on NEB digestion interruption) obtained by the library construction and sequencing method of the present example. Table 6 indicates that the human sample WGS PCR-free library (based on NEB digestion interruption) has relatively high sequencing quality on the high-throughput sequencing platform MGISEQ-2000RS PE150, which is self-developed by MGI.

**[Table 6] Sequencing quality of the human sample WGS PCR-free library (based on NEB digestion interruption)**

| | Acceptance level | NEB digestion interruption (PE150) | Covaris interruption (PE100) |
|---|---|---|---|
| Insert size of main band (bp) | | 419 | 405 |
| Clean read1 Q30 (%) | | 86.91 | 89.71 |
| Clean read2 Q30 (%) | >80 | 81.46 | 87.57 |
| Clean Q20 (%) | | 94.63 | 96.645 |
| Clean Q30 (%) | | 84.185 | 88.64 |
| GC content (%) | 39-42 | 40.88 | 41.05 |
| read 1 (AT) | <0.5% | 0.33 | 0.06 |
| read 1 (CG) | - | 0.38 | 0.36 |
| read 2 (AT) | <1% | 0.85 | 0.58 |
| read 2 (CG) | <1% | 0.58 | 0.54 |
| Mapping rate (%) | >98 | 98.84 | 99.17 |
| Unique rate (%) | >93 | 98.97 | 98.82 |
| Duplicate rate (%) | <3 | 1.03 | 1.18 |
| Average seq depth (X) | 30 | 30.19 | 30.33 |
| Coverage (%) | >99 | 99.07 | 99.09 |
| Coverage at least 20X (%) | >90 | 93.38 | 94.21 |
| Chimerical rate (%) | | 0.88 | 0.99 |
| Coverage bias Low Dropout | | 0.0490 | 0.0419 |
| Coverage bias High Dropout | | 0.0449 | 0.0364 |

Note: Covaris interruption (PE100) serves as a comparative PCR-free example, using the same library construction system, indicating that the library construction based on digestion interruption has the same effects as the library construction based on physical interruption.

Table 7 shows SNP and Indel variation detection and analysis results of the NA12878 WGS PCR-free library obtained by the library construction and sequencing method of the present example. Table 7 indicates that the PCR-free library (based on NEB digestion interruption) of the present disclosure is significantly superior to the PCR library in terms of Indel calling, and its overall performance is similar to NovaSeq PCR-free PE150 on the Illumina platform.

**[Table 7] SNP and Indel variation detection and analysis results of the NA12878 WGS PCR-free library (based on NEB digestion interruption)**

| | Acceptance level | NEB digestion interruption (PE150) | Covaris interruption (PE100) | BGISEQ-500 physical PCR (PE100) | NovaSeq PCR free (PE150) |
|---|---|---|---|---|---|
| snp_True-pos-call | | 3190000 | 3E+06 | 3E+06 | 3E+06 |
| snp_False-pos | | 4340 | 6650 | 5464 | 2045 |
| snp_False-neg | | 19000 | 17600 | 28154 | 8809 |
| snp_Precision | >0.995 | 0.9986 | 0.9979 | 0.9983 | 0.9994 |
| snp_Sensitivity | >0.99 | 0.9941 | 0.9945 | 0.9912 | 0.9973 |
| snp_F-measure | | 0.9963 | 0.9962 | 0.9947 | 0.9983 |
| indel_True-pos-call | | 469000 | 470000 | 457664 | 473679 |
| indel_False-pos | | 5920 | 5200 | 24056 | 4732 |
| indel_False-neg | | 11900 | 11700 | 23603 | 7588 |
| indel_Precision | >0.98 | 0.9876 | 0.9891 | 0.9501 | 0.9901 |
| indel_Sensitivit y | >0.97 | 0.9753 | 0.9757 | 0.951 | 0.9842 |
| indel_F-measure | | 0.9814 | 0.9823 | 0.9505 | 0.9872 |

Note: Covaris interruption (PE100) serves as a comparative PCR-free example, using the same library construction system, indicating that the library construction based on digestion interruption has the same effects as the library construction based on physical interruption. BGISEQ-500 physical PCR (PE100) serves as a PCR-based comparative example.

### Example 2 Construction and sequencing of 20 cfDNA libraries

Experimental objective: a plasma sample library was constructed by using an MGI PCR-free kit.

Sources of experimental samples: 20 plasma samples, including 2 abnormal chromosome samples.

### 1. Sample collection and treatment

2 mL of vein blood was collected and centrifuged at 1,600 g and 4°C for 10 min to separate blood cells from plasma, and the plasma was centrifuged at 16,000 g and 4°C for 10 min to further remove residual white blood cells. DNA was extracted from 200 µL of plasma and dissolved in 40 µL of TE solution.

### 2. End repair and addition of adenylate deoxyribonucleic acid

An end repair-A-tailing reaction solution was prepared according to Table 8.

**[Table 8] Composition of an end repair-A-tailing reaction solution**

| | |
|---|---|
| 10× T4 polynucleotide kinase buffer (Enzematics) | 5 µL |
| T4 polynucleotide kinase (10 U/µL) (Enzematics) | 1 µL |
| Mixed desoxyribonucleic acid solution (25 mM each) (Enzematics) | 0.5 µL |
| Taq DNA polymerase (5 U/µL) (Takara) | 0.4 µL |
| Adenylate deoxyribonucleic acid (100 mM) (Enzematics) | 0.5 µL |
| T4 DNA polymerase (3 U/µL) (Enzymatics) | 2 µL |
| Enzyme-free water (Sigma) | 0.6 µL |
| Total volume | 10 µL |

10 µL of prepared end repair-A-tailing reaction solution was added to and uniformly mixed with 40 µL of DNA, and the mixture was incubated at 37°C for 10 min and at 72°C for 15 min, and cooled to 4°C at a rate of 0.1s.

### 3. Ligation of an adapter

An adapter sequence used in the present protocol is as follows (in the present example, the 5'-end of the sequence is on the left side, the 3'-end of the sequence is on the right side, "//" represents a modifying group, "phos" represents phosphorylation, and the underlined bases represent a barcode of 10 bases).

### Phosphorylated adapter B strand:

/Phos/GAACGACATGGCTACGATCCGACTT (SEQ ID NO: 1); and
phosphorylated adapter T strand: 
/Phos/AGTCGGAGGCCAAGCGGTCTTAGGAAGACAANNNNNNNNNNCAACTCCTT
GGCTCACA (SEQ ID NO: 2),
 where N may be A, T, C or G.

Preparation of adapter: 20 µL of adapter B strands (100 µM), 20 µL of adapter T strands (100 µM), and 40 µL of 2× adapter buffer (components: 50 mM Tris-HCl (pH=8.0), 0.1 mM EDTA, and 50 mM NaCl) were mixed to prepare adapters A (25 µM). The adapters A were placed at the room temperature for more than half an hour and diluted to a use concentration or stored at -20°C. Before use, the adapters A (25 µM) were diluted with TE to prepare adapters B (1 µM).

1 µL of prepared adapters B (1 µM) was added to and thoroughly mixed with the product of step 3.

A ligation reaction solution was prepared according to Table 9.

**[Table 9] Composition of a ligation reaction solution**

| Component | Amount |
|---|---|
| 10× T4 PNK buffer (Enzymatics) | 3 µL |
| 0.1 M ATP (Thermo) | 0.8 µL |
| 50% PEG8000 (Rigaku) | 16 µL |
| T4 DNA ligase (600 U/µL) (Enzymatics) | 5 µL |
| Enzyme-free water (Sigma) | 4.2 µL |
| Total volume | 29 µL |

The prepared ligation reaction solution was uniformly vortex-mixed with the mixture of the adapters B and the product of step 4, and the mixture was subjected to transient centrifugation. The reaction sample was placed into the PCR instrument for reaction, the reaction conditions were as follows: 25°C for 30 min and 4°C hold, and the heated lid of the PCR instrument was set to 30°C. After the reaction was completed, 20 µL of TE buffer was added, 50 µL of XP magnetic beads (Beckman Coulter) was added for purification, and the collected product was dissolved in 22 µL of TE buffer. 15 µL of the respective samples was taken, multiple samples of the same volume were mixed and purified by adding XP magnetic beads (Beckman Coulter) in twice the volume of the sample mixture, and the collected product was dissolved in 22 µL of TE buffer.

### 4. Sequencing

A DNA nanoball can be prepared by multiple methods.

Method 1: referring to steps 6 to 9 of the whole genome library construction and sequencing of Example 1, single-strand cyclization of a sample, digestion of a linear single strand, preparation of DNA nanoballs, and sequencing on BGISEQ-500SE50+10 are performed. The sequencing and data analysis follow the standard operating process of BGISEQ-500 SE50+10.

Method 2: the constructed ligation product is taken and subjected to one-step preparation of DNA nanoballs and sequencing on BGISEQ-500SE50+ 10. The sequencing and data analysis follow the standard operating process of BGISEQ-500 SE50+10. In the present example, the method 2 was adopted.

### 5. Library construction and sequencing results of the present example

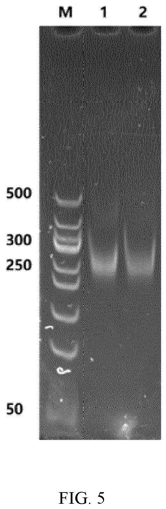

1 µL of ligation product was taken and subjected to PCR, and the size of an adapter-ligated fragment was verified. Results are shown in FIG. 5. It can be seen that, the size of the adapter-ligated PCR product is about 250 bp, which is in line with the theoretical value. The theoretical value is calculated in such a manner that the size of the cfDNA fragment is 160 bp, the overall length of the adapter is about 84 bp, and the overall length of the adapter-ligated fragment is about 260 bp.

Concentration detection results of the products in the respective steps are shown in Table 10. It can be seen that this method can be used to prepare a library from this type of sample.

**[Table 10] Concentration detection results of the products in the respective steps**

| | | |
|---|---|---|
| Concentration of the ligation product | 0.32 | ng/µL |
| Total mass of the ligation product | 12.8 | ng |
| Concentration of the DNA nanoball | 12.3 | ng/µL |

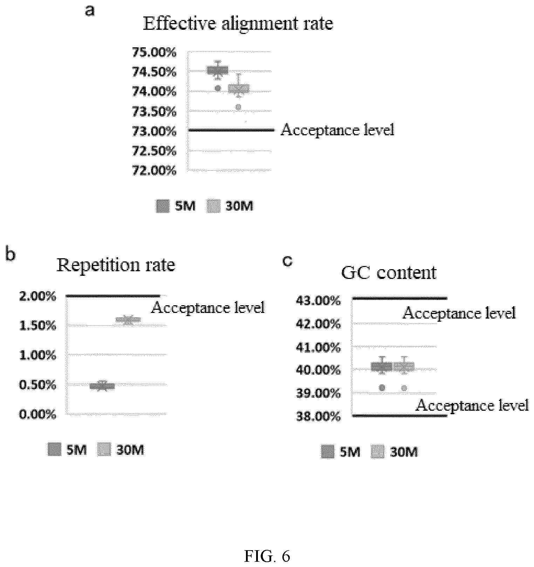

FIG. 6 shows analysis and statistical results of 5 MB and 30 MB of sequencing data. It can be seen that: in diagram a, effective alignment rates of all samples satisfy the requirements of the MGI prenatal test kit; in diagram b, repetition rates of all samples meet the requirements of the MGI prenatal test kit; and in diagram C, GC contents of all samples meet the requirements of the MGI prenatal test kit.

### Example 3 Construction of a human whole genome library with a self-developed platform PCR-free library construction kit (based on digestion interruption)

Experimental objective: a whole genome library was prepared from a human gDNA sample by using an MGI PCR-free kit in combination with an NEB digestion interruption kit.

Sources of experimental samples: NA12878 standard DNA (catalog number: NA12878, manufacturer: CORIELL INSTITUTE).

### 1. Digestion interruption, end repair, and A-tailing of the DNA sample

1 µg of standard DNA (dissolved in TE) was placed into each tube, interrupted with dsDNA Fragmentase, and subjected to end repair and A-tailing, and the volume of an interruption system was 50 µL. A corresponding reagent was thawed in advance and uniformly mixed, an enzyme reagent was uniformly mixed in an upside-down manner and placed on ice. A reaction system was prepared according to Table 11 on ice.

**[Table 11] Interruption and end repair and A-tailing reaction system for DNA sample**

| Component | Amount |
|---|---|
| 10× Fragmentase Reaction Buffer (NEB, M0348S) | 5 µL |
| Fragmentase (NEB, M0348S) | 3 µL |
| dATP (100 mM) (Enzymatics) | 1.7 µL |
| dNTPs (each 25 mM) (Enzymatics) | 2.3 µL |
| T4 DNA polymerase (3 U/µL) (Enzymatics) | 1 µL |
| rTaq (5 U/µL) (Enzymatics) | 1 µL |
| gDNA (dissolved in TE) | X µL |
| TE | Y µL |
| Total volume | 50 µL |

The DNA sample was added to and uniformly mixed with the prepared reaction system by pipetting or vortex-mixing; the mixture was subjected to transient centrifugation and immediately placed into the thermocycler for reaction, the reaction conditions were as followings: 4°C forever; 37°C for 20 min; 65°C for 30 min; and 4°C forever, and the heated lid of the PCR instrument was set to 70°C. After the reaction was completed, the sample was collected and placed on ice immediately, and TE was added to make up the volume of the sample to 50 µL.

### 2. Selection of a DNA fragment

(1) 100 µL of interrupted sample was taken and transferred into a new 1.5 mL non-stick tube; 60 µL of XP magnetic beads was added to and uniformly mixed with the sample by shaking, and allowed to bind to DNA at the room temperature for 10 min; the tube was placed onto the magnetic rack; the magnetic beads were allowed to bind to DNA for 2 min (until the liquid became clear); and the supernate was carefully taken by suction and transferred into a new 1.5 mL EP tube (the supernate was reserved at this step). 15 µL of XP magnetic beads was added to and uniformly mixed with the supernate by shaking, and allowed to bind to DNA at the room temperature for 10 min; the tube was placed onto the magnetic rack, the magnetic beads were allowed to bind to DNA for 2 min (until the liquid became clear); and the supernate was removed by suction.
(2) 500 µL of 75% ethanol was placed into the non-stick tube on the magnetic rack, the tube cap was closed, the mixture in the tube was uniformly mixed, and the supernate was removed. After washing with 500 µL of 75% ethanol again, residual ethanol was removed as much as possible by using a pipette with a small measurement range, and the magnetic beads were air-dried at the room temperature.
(3) The magnetic beads were resuspended and uniformly mixed with 42 µL of TE by shaking, and allowed to bind to DNAs at the room temperature for 10 min; the tube was placed onto the magnetic rack, the magnetic beads were allowed to bind to DNAs for 2 min (until the liquid became clear); and 40 µL of supernate was carefully taken by suction and transferred into a new 1.5 mL EP tube for next reaction, or it stored in a refrigerator at -20°C.

### 3. Quantitation and normalization of the sample

2 µL of purified DNA was taken and subjected to Qubit dsDNA HS quantitation. The selected DNA fragments were normalized according to the concentration determined by Qubit quantitation; the mass of the DNA fragment was adjusted to 150 ng; and 1× TE was added to make up the total volume of 40 µL. If necessary, the normalized samples can be stored in a refrigerator at -20°C.

The size of the obtained DNA fragment was 300 bp to 500 bp.

### 5. Ligation of adapter

An adapter sequence used in the present protocol is as follows (in the present example, the 5'-end of the sequence is on the left side, the 3'-end of the sequence is on the right side, "//" represents a modifying group, "phos" represents phosphorylation, and the underlined bases represent a barcode of 10 bases).

### Phosphorylated adapter B strand:

/Phos/GAACGACATGGCTACGATCCGACTT (SEQ ID NO: 1); and
phosphorylated adapter T strand: 
/Phos/AGTCGGAGGCCAAGCGGTCTTAGGAAGACAANNNNNNNNNNCAACTCCTT
GGCTCACA (SEQ ID NO: 2),
 where N may be A, T, C or G.

Preparation of the adapter: 20 µL of adapter B strands (100 µM), 20 µL of adapter T strands (100 µM), and 40 µL of 2× adapter buffer (components: 50 mM Tris-HCl (pH=8.0), 0.1 mM EDTA, and 50 mM NaCl) were mixed to prepare adapters A (25 µM); and the adapters A were placed at the room temperature for more than half an hour and then diluted to a use concentration or stored at -20°C. Before use, the adapters A (25 µM) were diluted with TE to prepare adapters B (6 µM).

5 µL of the prepared adapters B (6 µM) was added to and thoroughly mixed with the product of step 4.

A ligation reaction solution was prepared according to Table 12.

**[Table 12] Composition of a ligation reaction solution**

| Component | Amount |
|---|---|
| 10× T4 PNK buffer (Enzymatics) | 3 µL |
| 0.1 M ATP (Thermo) | 0.8 µL |
| 50% PEG8000 (Rigaku) | 16 µL |
| T4 DNA ligase (600 U/µL) (Enzymatics) | 5 µL |
| Enzyme-free water (Sigma) | 0.2 µL |
| Total volume | 25 µL |

The prepared ligation reaction solution was uniformly vortex-mixed with the mixture of the adapters B and the product of step 4, and the mixture was subjected to transient centrifugation. The reaction sample was placed into the PCR instrument for reaction, the reaction conditions were as follows: 25°C for 30 min; and 4°C hold, and the heated lid of the PCR instrument was set to 30°C. After the reaction was completed, 20 µL of TE buffer was added, 50 µL of XP magnetic beads was added for purification, and the collected product was dissolved in 50 µL of TE buffer.

### 6. Single-strand cyclization

48 µL of purified product was incubated at 95°C for 3 min and at 4°C for 10 min.

A single-strand cyclization reaction solution was prepared according to Table 13.

**[Table 13] Composition of a single-strand cyclization reaction solution**

| Component | Amount |
|---|---|
| 10× TA buffer (Epicentre) | 6 µL |
| 100 mM ATP (Thermo) | 0.6 µL |
| 20 µM mediation fragments | 2.5 µL |
| T4 DNA ligase (600 U/µL) (Enzymatics) | 1 µL |
| Enzyme-free water (Sigma) | 1.9 µL |
| Total volume | 12 µL |

12 µL of the prepared single-strand cyclization reaction solution was uniformly vortex-mixed with 48 µL of thermal denaturation product, and the mixture was subjected to transient centrifugation. The reaction sample was placed into the PCR instrument for reaction, the reaction conditions were as follows: 37°C for 60 min; and 4°C hold, and the heated lid of the PCR instrument was set to 42°C.

20 µM fragments for mediation have a corresponding complementary sequence to be ligated to both ends of the single strand. The corresponding complementary sequence is (in the present example, the 5'-end of the sequence is on the left side, and the 3'-end of the sequence is on the right side): GCCATGTCGTTCTGTGAGCCAAGG (SEQ ID NO: 8).

### 7. Digestion of a linear single strand

A digestion reaction solution was prepared according to Table 14.

**[Table 14] Composition of a digestion reaction solution**

| Component | Amount |
|---|---|
| 10× TA buffer (Epicentre) | 0.4 µL |
| ExoI (20 U/µL) (Enzymatics) | 2 µL |
| ExoIII (10 U/µL) (Enzymatics) | 1 µL |
| Enzyme-free water | 0.6 µL |
| Total volume | 4 µL |

4 µL of prepared digestion reaction solution was added to and uniformly mixed with 60 µL of reaction product of the previous step. The mixture was incubated at 37°C for 30 min, and added and uniformly mixed with 3 µL of EDTA (500 mM, Ambion). A product was purified and collected with 120 µL of XP magnetic beads, and dissolved in 30 µL of TE buffer.

### 8. Quantitation of a single-stranded circle

The single-stranded cyclization product obtained through the digestion of the linear single strand in the previous step was quantitated by using a Qubit ssDNA Assay Kit.

Concentration detection results of the products in the respective steps are shown in Table 15. It can be seen that, the integration of the interruption, end repair, and A-tailing in one step is also suitable for the PCR-free library construction.

**[Table 15] Concentration detections results of the products in the respective steps**

| | | |
|---|---|---|
| Concentration of the selected fragment | 2.6 | ng/µL |
| Total mass of the selected fragment | 130 | ng |
| Concentration of the single-stranded circle | 2.16 | ng/µL |

### Example 4 Construction of a human whole genome library with a self-developed platform PCR-free library construction kit (based on digestion interruption)

Experimental objective: a whole genome library was prepared from a human gDNA sample by using an MGI PCR-free kit in combination with an NEB digestion interruption kit.

Sources of experimental samples: NA12878 standard DNA (catalog number: NA12878, manufacturer: CORIELL INSTITUTE).

### 1. Digestion interruption and end repair of the DNA sample

1 µg of standard DNA (dissolved in TE) was placed into each tube, interrupted with dsDNA Fragmentase (catalog number: M0348, NEB), and subjected to end repair, and the volume of the system was 50 µL. 10× Fragmentase Reaction Buffer v2 was thawed in advance and uniformly vortex-mixed, dsDNA Fragmentase was uniformly vortex-mixed and placed on ice. A reaction system was prepared according to Table 16 on ice.

**[Table 16] Digestion interruption and end repair reaction system for the DNA sample**

| Component | Amount |
|---|---|
| 10× Fragmentase Reaction Buffer v2 (NEB) | 5 µL |
| dsDNA Fragmentase (NEB) | 3 µL |
| dNTPs (each 25 mM) (Enzymatics) | 3 µL |
| DNA polymerase I (10 U/µL) (NEB) | 2 µL |
| 1 M MgCl₂ (Sigma) | 0.3 µL |
| Enzyme-free water (Sigma) | 6.7 µL |
| Total volume | 20 µL |

The above reaction system was uniformly pipetted, 20 µL of gDNA sample (total mass was 1 ug) was added to and uniformly mixed with the reaction system by gently pipetting 6-8 times or vortex-mixing; the mixture was subjected to transient centrifugation and immediately placed into the thermocycler for reaction; the reaction conditions were as follows: 37°C for 30 min; and 4°C forever, and the heated lid of the PCR instrument was set to 70°C. After the reaction was completed, the sample was collected and placed on ice immediately, and TE was added to make up the volume of the sample to 30 µL.

### 2. Selection of a DNA fragment

(1) 100 µL of interrupted sample was taken and transferred into a new 1.5 mL non-stick tube; 52 µL of XP magnetic beads was added to and uniformly mixed with the sample by shaking, and allowed to bind to DNA at the room temperature for 10 min; after transient centrifugation, the tube was placed onto the magnetic rack, the magnetic beads were allowed to bind to DNA for 2 min (until the liquid became clear); and the supernate was carefully taken by suction and transferred into a new 1.5 mL EP tube (the supernate was reserved at this step). 15 µL of XP magnetic beads was added to and uniformly mixed with the supernate by shaking, and allowed to bind to DNA at the room temperature for 10 min; the tube was placed onto the magnetic rack, the magnetic beads were allowed to bind to DNA for 2 min (until the liquid became clear); and the supernate was removed by suction.
(2) 500 µL of 75% ethanol was placed into the non-stick tube on the magnetic rack, the tube cap was closed, the mixture in the tube was uniformly mixed, and the supernate was removed. After washing with 500 µL of 75% ethanol again, residual ethanol was removed as much as possible by using a pipette with a small measurement range, and the magnetic beads were air-dried at the room temperature.
(3) The magnetic beads were resuspended in and uniformly mixed with 42 µL of TE by shaking, and allowed to bind to DNA at the room temperature for 10 min; after transient centrifugation, the tube was placed onto the magnetic rack, the magnetic beads were allowed to bind to DNA for 2 min (until the liquid became clear); and 40 µL of supernate was carefully taken by suction and transferred into a new 1.5 mL EP tube for the next reaction, or it was stored in a refrigerator at -20°C.

### 3. Quantitation and normalization of the sample

2 µL of purified DNA was taken and subjected to Qubit dsDNA HS quantitation. The selected DNA fragment was normalized according to the concentration determined by Qubit quantitation; the mass of the DNA fragment was adjusted to 150 ng; and 1× TE was added to make up the total volume of 40 µL. If necessary, the normalized samples can be stored in a refrigerator at -20°C.

The size of the obtained DNA fragment was 300 bp to 500 bp.

### 4. A-tailing

First, an A-tailing reaction solution was prepared according to Table 17.

**[Table 17] Composition of an A-tailing reaction solution**

| Component | Amount |
|---|---|
| T4 10× PNK buffer (Enzymatics) | 5 µL |
| dATP (100 mM) (Enzymatics) | 0.5 µL |
| dNTPs (each 25 mM) (Enzymatics) | 0.35 µL |
| rTaq (5 U/µL) (Enzymatics) | 0.2 µL |
| Enzyme-free water (Sigma) | 1 µL |

10 µL of prepared A-tailing reaction solution was added to and vortex-mixed with 40 µL of product of step 3; the mixture was subjected to transient centrifugation; and the total volume of the mixture was made up to 50 µL. The reaction sample was placed into the PCR instrument for reaction, the reaction conditions were as follows: 65°C for 30 min; and 4°C forever, and the heated lid of the PCR instrument was set to 70°C.

### 5. Ligation of adapter

An adapter sequence used in the present protocol is as follows (in the present example, the 5'-end of the sequence is on the left side, the 3'-end of the sequence is on the right side, "//" represents a modifying group, "phos" represents phosphorylation, and the underlined bases represent a barcode of 10 bases).

### Phosphorylated adapter B strand:

/Phos/GAACGACATGGCTACGATCCGACTT (SEQ ID NO: 1); and
phosphorylated adapter T strand: 
/Phos/AGTCGGAGGCCAAGCGGTCTTAGGAAGACAANNNNNNNNNNCAACTCCTT
GGCTCACA (SEQ ID NO: 2),
 where N may be A, T, C or G.

Preparation of the adapter: 20 µL of adapter B strands (100 µM), 20 µL of adapter T strands (100 µM), and 40 µL of 2× adapter buffer (components: 50 mM Tris-HCl (pH=8.0), 0.1 mM EDTA, and 50 mM NaCl) were mixed to prepare adapters A (25 µM); and the adapters A were placed at the room temperature for more than half an hour and then diluted to a use concentration or stored at -20°C. Before use, the adapters A (25 µM) were diluted with TE to prepare adapters B (6 µM).

5 µL of prepared adapters B (6 µM) was added to and thoroughly mixed with the product of step 4.

A ligation reaction solution was prepared according to Table 18.

**[Table 18] Composition of a ligation reaction solution**

| Component | Amount |
|---|---|
| 10× T4 PNK buffer (Enzymatics) | 3 µL |
| 0.1 M ATP (Thermo) | 0.8 µL |
| 50% PEG8000 (Rigaku) | 16 µL |
| T4 DNA ligase (600 U/µL) (Enzymatics) | 5 µL |
| Enzyme-free water (Sigma) | 0.2 µL |
| Total volume | 25 µL |

The prepared ligation reaction solution was uniformly vortex-mixed with the mixture of the adapters B and the product of step 4, and the mixture was subjected to transient centrifugation. The reaction sample was placed into the PCR instrument for reaction, the reaction conditions were as follows: 25°C for 30 min; and 4°C hold, and the heated lid of the PCR instrument was set to 30°C. After the reaction was completed, 20 µL of TE buffer was added, 50 µL of XP magnetic beads was added for purification, and the collected product was dissolved in 50 µL of TE buffer.

### 6. Single-strand cyclization

5 µL of fragments (20 µM) for mediation and 2.5 µL of NaOH (2 M, Sigma) were added to and uniformly vortex-mixed with 48 µL of purified product, and the mixture was placed at the room temperature for 5 min. 5 µL of Tris-HCl (1 M, pH=6.8) was added to and uniformly vortex-mixed with the mixture, and a single-strand cyclization reaction solution shown in Table 19 was added.

**[Table 19] Composition of a single-strand cyclization reaction solution**

| Component | Amount |
|---|---|
| 10× TA buffer (Epicentre) | 6 µL |
| 100 mM ATP (Thermo) | 0.6 µL |
| T4 DNA ligase (600 U/µL) (Enzymatics) | 0.4 µL |
| Total volume | 7 µL |

The reaction sample was placed into the PCR instrument for reaction, the reaction conditions were as follows: 37°C for 30 min; and 4°C hold, and the heated lid of the PCR instrument was set to 42°C.

20 µM fragments for mediation have a corresponding complementary sequence to be ligated to both ends of the single strand. The corresponding complementary sequence is (in the present example, the 5'-end of the sequence is on the left side, and the 3'-end of the sequence is on the right side): GCCATGTCGTTCTGTGAGCCAAGG (SEQ ID NO: 8).

### 7. Digestion of a linear single strand

A digestion reaction solution was prepared according to Table 20.

**[Table 20] Composition of a digestion reaction solution**

| Component | Amount |
|---|---|
| 10× TA buffer (Epicentre) | 0.4 µL |
| ExoI (20 U/µL) (Enzymatics) | 2 µL |
| ExoIII (10 U/µL) (Enzymatics) | 1 µL |
| Enzyme-free water | 0.6 µL |
| Total volume | 4 µL |

4 µL of prepared digestion reaction solution was added to and uniformly mixed with 67.5 µL of reaction product of the previous step. The mixture was incubated at 37°C for 30 min, and added and uniformly mixed with 3 µL of EDTA (500 mM, Ambion). A product was purified and collected with 120 µL of XP magnetic beads, and dissolved in 30 µL of TE buffer.

### 8. Quantitation of a single-stranded circle

The single-stranded cyclization product obtained through the digestion of the linear single strand in the previous step was quantitated by using a Qubit ssDNA Assay Kit.

Concentration detection results of the products in the respective steps are shown in Table 21. It can be seen that, the integration of the interruption, end repair, and A-tailing in one step is also suitable for PCR-free library construction.

**[Table 21] Concentration detection results of the products in the respective steps**

| | | |
|---|---|---|
| Concentration of the selected fragment | 4.6 | ng/µL |
| Total mass of the selected fragment | 184 | ng |
| Concentration of the single-stranded circle | 2.2 | ng/µL |

**Example 5** Construction of a human whole genome library with a self-developed PCR-free library construction kit (based on digestion interruption) in combination with a dual-barcode adapter, and sequencing thereof

Experimental objective: a whole genome library was prepared from a human gDNA sample by using an MGI PCR-free kit in combination with a dual-barcode adapter and an NEB digestion interruption kit.

Sources of experimental samples: NA12878 standard DNA (catalog number: NA12878, manufacturer: CORIELL INSTITUTE).

### 1. Digestion interruption of the DNA sample

1 µg of standard DNA (dissolved in TE) was placed into each tube and interrupted with dsDNA Fragmentase (catalog number: M0348, NEB), the volume of an interruption system was 50 µL. 10× Fragmentase Reaction Buffer v2 was thawed in advance and uniformly vortex-mixed, and dsDNA Fragmentase was uniformly vortex-mixed and placed on ice. A reaction system was prepared according to Table 22 on ice.

**[Table 22] Digestion interruption reaction system for DNA sample**

| Component | Amount |
|---|---|
| 10× Fragmentase Reaction Buffer v2 (NEB) | 3 µL |
| gDNA (dissolved in TE) | X µL |
| TE | 27-X µL |
| Total volume | 27 µL |

The sample, after being pipetted, was added with and gently uniformly mixed with 3 µL of dsDNA Fragmentase by pipetting 6-8 times or vortex-mixing. After transient centrifugation, the mixture was immediately placed into the thermocycler for reaction, the reaction conditions were as follows: 37°C for 25 min; 65°C for 15 min; and 4°C forever, and the heated lid of the PCR instrument was set to 70°C. After the reaction was completed, the sample was collected and placed on ice immediately, and TE was added to make up the total volume of the sample to 70 µL.

### 2. Selection of a DNA fragment

(1) 100 µL of interrupted sample was taken and transferred into a new 1.5 mL non-stick tube; 60 µL of XP magnetic beads was added to and uniformly mixed with the sample by shaking, and allowed to bind to DNA at the room temperature for 10 min; after transient centrifugation, the tube was placed onto the magnetic rack, the magnetic beads were allowed to bind to DNA for 2 min (until the liquid became clear); and the supernate was carefully taken by suction and transferred into a new 1.5 mL EP tube (the supernate was reserved at this step). 15 µL of XP magnetic beads was added to and uniformly mixed with the supernate by shaking, and allowed to bind to DNA at the room temperature for 10 min. The tube was placed onto the magnetic rack, the magnetic beads were allowed to bind to DNA for 2 min (until the liquid became clear), and the supernate was removed by suction.
(2) 500 µL of 75% ethanol was placed into the non-stick tube on the magnetic rack, the tube cap was closed, the mixture in the tube was uniformly mixed. The supernate was removed. After washing with 500 µL of 75% ethanol again, residual ethanol was removed as much as possible by using a pipette with a small measurement range, and the magnetic beads were air-dried at the room temperature.
(3) The magnetic beads were resuspended in and uniformly mixed with 42 µL of TE by shaking, and allowed to bind to DNA at the room temperature for 10 min; after transient centrifugation, the tube was placed onto the magnetic rack, the magnetic beads were allowed to bind to DNA for 2 min (until the liquid became clear); and 40 µL of supernate was carefully taken by suction and transferred into a new 1.5 mL EP tube for next reaction, or it was stored in a refrigerator at -20°C.

### 3. Quantitation and normalization of the sample

2 µL of purified DNA was taken and subjected to Qubit dsDNA HS quantitation. The selected DNA fragment was normalized according to the concentration determined by Qubit quantitation, the mass of the DNA fragment was adjusted to 150 ng, and 1× TE was added to make up the total volume of 40 µL. If necessary, the normalized samples can be stored in a refrigerator at -20°C.

The size of the obtained DNA fragment was 300 bp to 500 bp.

### 4. End repair and A-tailing

First, an end repair-A-tailing reaction solution was prepared according to Table 23.

**[Table 23] Composition of an end repair-A-tailing reaction solution**

| Component | Amount |
|---|---|
| T4 10× PNK buffer (Enzymatics) | 5 µL |
| dATP (100 mM) (Enzymatics) | 0.5 µL |
| dNTPs (each 25 mM) (Enzymatics) | 0.5 µL |
| T4 DNA polymerase (3 U/µL) (Enzymatics) | 2 µL |
| T4 PNK (10 U/µL) (Enzymatics) | 1 µL |
| rTaq (5 U/µL) (Enzymatics) | 1 µL |
| Enzyme-free water (Sigma) | 1 µL |

10 µL of prepared end repair-A-tailing reaction solution was added to and uniformly vortex-mixed with 40 µL of product of step 3; the mixture was subjected to transient centrifugation; and the total volume of the mixture was made up to 50 µL. The reaction sample was placed into the PCR instrument for reaction, the reaction conditions were as follows: 14°C for 15 min; 37°C for 25 min; 65°C for 15 min; and 4°C forever, and the heated lid of the PCR instrument was set to 70°C.

### 5. Ligation of an adapter

An adapter sequence used in the present protocol is as follows (in the present example, the 5'-end of the sequence is on the left side, the 3'-end of the sequence is on the right side, "//" represents a modifying group, "phos" represents phosphorylation, and the underlined bases represent a barcode of 10 bases).

### Phosphorylated adapter B strand:

/Phos/TCTCAGTACGTCAGCAGTTNNNNNNNNNNCAACTCCTTGGCTCACAGAACG
ACATGGCTACGATCCGACTT (SEQ ID NO: 7);

and phosphorylated adapter T strand: 
/Phos/AGTCGGAGGCCAAGCGGTCTTAGGAAGACAANNNNNNNNNNCTGATAAGG

TCGCCATGCC (SEQ ID NO: 6),
 where N may be A, T, C or G.

Preparation of the adapter: 20 µL of adapter B strands (100 µM), 20 µL of adapter T strands (100 µM), and 40 µL of 2× adapter buffer (components: 50 mM Tris-HCl (pH=8.0), 0.1 mM EDTA, and 50 mM NaCl) were mixed to prepare adapters A (25 µM); and the adapters A were placed at the room temperature for more than half an hour and then diluted to a use concentration or stored at -20°C. Before use, the adapters A (25 µM) were diluted with TE to prepare adapters B (6 µM).

5 µL of the prepared adapters B (6 µM) was added to and thoroughly mixed with the product of step 4.

A ligation reaction solution was prepared according to Table 24.

**[Table 24] Composition of a ligation reaction solution**

| Component | Amount |
|---|---|
| 10× T4 PNK buffer (Enzymatics) | 3 µL |
| 0.1 M ATP (Thermo) | 0.8 µL |
| 50% PEG8000 (Rigaku) | 16 µL |
| T4 DNA ligase (600 U/µL) (Enzymatics) | 5 µL |
| Enzyme-free water (Sigma) | 0.2 µL |
| Total volume | 25 µL |

The prepared ligation reaction solution was uniformly vortex-mixed with the mixture of the adapters B and the product of step 4, and the mixture was subjected to transient centrifugation. The reaction sample was placed into the PCR instrument for reaction, the reaction conditions were as follows: 25°C for 30 min; and 4°C hold, and the heated lid of the PCR instrument was set to 30°C. After the reaction was completed, 20 µL of TE buffer was added, 50 µL XP magnetic beads was added for purification, and the collected product was dissolved in 50 µL of TE buffer.

### 6. Single-strand cyclization

48 µL of purified product was incubated at 95°C for 3 min and at 4°C for 10 min.

A single-strand cyclization reaction solution was prepared according to Table 25.

**[Table 25] Composition of a single-strand cyclization reaction solution**

| Component | Amount |
|---|---|
| 10× TA buffer (Epicenter) | 6 µL |
| 100 mM ATP (Thermo) | 0.6 µL |
| 20 µM mediation fragments | 2.5 µL |
| T4 DNA ligase (600 U/µL) (Enzymatics) | 1 µL |
| Enzyme-free water (Sigma) | 1.9 µL |
| Total volume | 12 µL |

12 µL of the prepared single-strand cyclization reaction solution was uniformly vortex-mixed with 48 µL of thermal denaturation product, and the mixture was subjected to transient centrifugation. The reaction sample was placed into the PCR instrument for reaction, the reaction conditions were as follows: 37°C for 60 min; and 4°C hold, and the heated lid of the PCR instrument was set to 42°C.

20 µM fragments for mediation have a corresponding complementary sequence to be ligated to both ends of the single strand. The corresponding complementary sequence is (in the present example, the 5'-end of the sequence is on the left side, and the 3'-end of the sequence is on the right side): TGCTGACGTACTGAGAGGCATGGCGACCT (SEQ ID NO: 8).

### 7. Digestion of a linear single strand

A digestion reaction solution was prepared according to Table 26.

**[Table 26] Composition of a digestion reaction solution**

| Component | Amount |
|---|---|
| 10× TA buffer (Epicenter) | 0.4 µL |
| ExoI (20 U/µL) (Enzymatics) | 2 µL |
| ExoIII (10 U/µL) (Enzymatics) | 1 µL |
| Enzyme-free water | 0.6 µL |
| Total volume | 4 µL |

4 µL of prepared digestion reaction solution was added to and uniformly mixed with 60 µL of reaction product of the previous step. The mixture was incubated at 37°C for 30 min, and added and uniformly mixed with 3 µL of EDTA (500 mM, Ambion). A product was purified and collected with 120 µL of XP magnetic beads, and dissolved in 30 µL of TE buffer.

### 8. Quantitation of a single-stranded circle

The single-stranded cyclization product obtained through the digestion of the linear single strand in the previous step was quantitated by using a Qubit ssDNA Assay Kit.

### 9. Sequencing

DNA nanoballs were prepared with the constructed single-stranded circular DNA library and sequenced on MGISEQ-2000 PE150. The sequencing and data analysis followed the standard operating process of MGISEQ-2000 PE150.

### 10. Library construction and sequencing results of the present example

Concentration detection results of the products in the respective steps are shown in Table 27. Table 28 shows the sequencing quality of the human sample WGS PCR-free library (based on digestion interruption) obtained by the library construction and sequencing method of the present example. Table 28 indicates that the human sample WGS PCR-free library (based on digestion interruption) has relatively high sequencing quality on the high-throughput sequencing platform MGISEQ-2000RS PE150, self-developed by MGI.

**[Table 27] Concentrations detection results of the products in the respective steps**

| | | |
|---|---|---|
| Concentration of the selected fragment | 3.35 | ng/µL |
| Total mass of the selected fragment | 150.75 | ng |
| Concentration of the single-stranded circle | 1.25 | ng/µL |

**[Table 28] Sequencing quality of the human sample WGS PCR-free library (based on digestion interruption) obtained by the library construction and sequencing method of the present example**

| | Acceptance level | Digestion interruption + dual-barcode adapter (PE150) | Covaris interruption (PE100) |
|---|---|---|---|
| Insert size of the main band (bp) | | 424 | 405 |
| Clean read1 Q30 (%) | | 97.4 | 89.71 |
| Clean read2 Q30 (%) | >80 | 97.63 | 87.57 |
| Clean Q20 (%) | | 93.37 | 96.645 |
| Clean Q30 (%) | | 93.33 | 88.64 |
| GC content (%) | 39-42 | 41.08 | 41.05 |
| read 1 (AT) | <0.5% | 0.22 | 0.06 |
| read_1 (CG) | - | 0.17 | 0.36 |
| read_2 (AT) | <1% | 0.42 | 0.58 |
| read_2 (CG) | <1% | 0.38 | 0.54 |
| Mapping rate (%) | >98 | 99.98 | 99.17 |
| Unique rate (%) | >93 | 99.41 | 98.82 |
| Duplicate rate (%) | <3 | 0.59 | 1.18 |
| Average seq depth (X) | 30 | 31 | 30.33 |
| Coverage (%) | >99 | 99.16 | 99.09 |
| Coverage at least 20X (%) | >90 | 93.73 | 94.21 |
| Chimerical rate (%) | | 1.62 | 0.99 |
| Coverage bias Low Dropout | | 0.0438 | 0.0419 |
| Coverage bias High Dropout | | 0.0408 | 0.0364 |

Note: Covaris interruption (PE100) serves as a comparative PCR-free example, and using the same library construction system, indicating that library construction based on digestion interruption combined with a dual-barcode adapter has the same effect as the library construction based on physical interruption.

Table 29 shows SNP and Indel variation detection and analysis results of the NA12878 PCR-free library (based on NEB digestion interruption) obtained by the library construction and sequencing method of the present example. Table 29 indicates that the PCR-free library (based on digestion interruption) of the present disclosure is significantly superior to the PCR library in terms of Indel calling, and its overall performance is similar to that of NovaSeq PCR-free PE150 on the Illumina platform.

**[Table 29] SNP and Indel variation detection and analysis results of the NA12878 WGS PCR-free library (based on digestion interruption)**

| | Acceptance level | Digestion interruption + dual-barcode adapter (PE150) | Covaris interruption (PE100) | NovaSeq PCR free (PE150) |
|---|---|---|---|---|
| snp_True-pos-call | | 3.19E+06 | 3.19E+06 | 3E+06 |
| snp_False-pos | | 1.87E+03 | 6.65E+03 | 2045 |
| snp_False-neg | | 1.94E+04 | 1.76E+04 | 8809 |
| snp_Precision | >0.995 | 0.9994 | 0.9979 | 0.9994 |
| snp_Sensitivity | >0.99 | 0.9939 | 0.9945 | 0.9973 |
| snp_F-measure | | 0.9967 | 0.9962 | 0.9983 |
| indel_True-pos-call | | 4.74E+05 | 4.70E+05 | 473679 |
| indel_False-pos | | 3.81E+03 | 5.20E+03 | 4732 |
| indel_False-neg | | 6.86E+03 | 1.17E+04 | 7588 |
| indel_Precision | >0.98 | 0.992 | 0.9891 | 0.9901 |
| indel_Sensitivity | >0.97 | 0.9857 | 0.9757 | 0.9842 |
| indel_F-measure | | 0.9889 | 0.9823 | 0.9872 |

Note: Covaris interruption (PE100) serves as a comparative PCR-free example, using the same library construction system, indicating that the library construction based on digestion interruption has the same effects as the library construction based on physical interruption.

### Industrial Application

The PCR-free construction solutions provided by the present disclosure overcomes the problems such as base pairing mistake, data bias, and repetitive sequences, which may be introduced by PCR during library construction. Furthermore, these solutions are compatible with library construction based on different interruption methods and small inputs. In combination with the nanoball preparation method based on rolling circle replication, the PCR-free library construction method of the present disclosure achieves true PCR-free library construction and sequencing for samples, thereby achieving whole PCR-free process. In the present disclosure, by adopting an optimized system for end repair and adapter ligation and the one-step reaction of single-strand cyclization and rolling circle replication, the library construction efficiency of the self-developed platform PCR-free library construction kit is improved, and required DNA inputs are reduced. The library construction system of the present disclosure is compatible with different types of starting samples, which include, but are not limited to, genomic DNA, interrupted DNA, DNA or an interrupted DNA product obtained by whole genome amplification, amplicon DNA, cfDNA, and DNA obtained by reverse transcription of RNA. Specifically, compared to the prior art, the present disclosure has the following advantages. 1) Wide applicability: the present disclosure is applicable to all species having known or unknown reference sequences; it can be adopted by general molecular biology laboratories; and it is compatible with library construction based on physical interruption and digestion interruption, compatible with different types of samples, which include, but are not limited to, genomic DNA, interrupted DNA, DNA or an interrupted DNA product obtained by whole genome amplification, amplicon DNA, cfDNA, and DNA obtained by reverse transcription of RNA. 2) Simple operation and shorter time for library construction: according to the present disclosure, the end repair and the A-tailing reaction are performed in the same tube; the magnetic bead purification is omitted to directly perform the adapter ligation; and the conventional PCR amplification and purification are omitted, thereby greatly shortening the time for library construction. Furthermore, according to the library construction method, the cyclization and the rolling circle replication are performed simultaneously, thereby further shortening the time for library construction. 3) High library construction efficiency: according to the library construction method, by adopting the optimized system for end repair and A-tailing, the optimized system for adapter ligation, and the one-step of cyclization and rolling circle replication reaction, a pooling library construction and sequencing with small inputs of starting samples as well as the PCR-free library construction using 200 µL of plasma DNA can be achieved. 4) Enhanced accuracy of sequencing data on the self-developed platform: the methods of the present disclosure achieve the true PCR-free library construction and sequencing, which can improve the accuracy and sensitivity of SNP and InDel detection, and the methods of the present disclosure especially have excellent performance in term of InDel detection over other platforms from business competitors, for example, Illumina.

## Claims

1. A PCR-free high-throughput sequencing method, comprising the following steps:
(A1) obtaining a DNA fragment of target size by performing or not performing, based on a size of a nucleic acid sample, fragmentation on the nucleic acid sample;
(A2) performing end repair and an A-tailing reaction on the product of step (A1);
(A3) ligating an adapter to the product of step (A2);
(A4) obtaining DNA nanoballs by performing single-strand cyclization on the product of step (A3) and rolling circle replication; and
(A5) loading and sequencing.

2. A PCR-free high-throughput sequencing method, comprising the following steps:
(B1) obtaining a DNA fragment of target size by performing fragmentation on a nucleic acid sample based on a size of the nucleic acid sample, and performing end repair and an A-tailing reaction;
(B2) ligating an adapter to the product of step (B 1);
(B3) obtaining DNA nanoballs by performing single-strand cyclization on the product of step (B2) and rolling circle replication; and
(B4) loading and sequencing.

3. A PCR-free high-throughput sequencing method, comprising the following steps:
(C1) performing fragmentation on a nucleic acid sample, based on a size of the nucleic acid sample, and performing end repair at the same time to obtain a DNA fragment of target size;
(C2) performing an A-tailing reaction on the product of step (C1);
(C3) ligating an adapter to the product of step (C2);
(C4) obtaining DNA nanoballs by performing single-strand cyclization on the product of step (C3) and rolling circle replication; and
(C5) loading and sequencing.

4. The method according to claim 3, wherein in step (B1), or (C1), the fragmentation is performed by digesting the nucleic acid sample with fragmentmase.

5. The method according to any one of claims 1 to 3, wherein the adapter comprises at least one barcode.

6. The method according to claim 5, wherein one adapter comprises two barcodes.

7. The method according to claim 6, wherein:
the adapter is formed by annealing two partially complementary single-stranded nucleic acids, and
the two barcodes are located in a non-complementary region of the two single-stranded nucleic acids.

8. The method according to any one of claims 1 to 3, wherein in step (A1), (B1), or (C1), the nucleic acid sample is DNA or RNA.

9. The method according to claim 8, wherein the DNA is genomic DNA, a naturally occurring small-molecule DNA, or an amplified DNA product.

10. The method according to claim 1, wherein in step (A1), the fragmentation is performed through physical interruption or digestion interruption.

11. The method according to claim 8, wherein, when the nucleic acid sample is RNA,
the RNA is subjected to reverse transcription to obtain DNA; and
the fragmentation is performed on the RNA or the DNA obtained by the reverse transcription of the RNA.

12. The method according to claim 1, wherein:
in step (A2), the end repair and the A-tailing reaction are performed in one step by mixing and reacting an end repair-A-tailing reaction solution with the product of step (A1), to obtain the product of step (A2); and
the end repair-A-tailing reaction solution contains a T4 polynucleotide kinase buffer, adenylate deoxyribonucleic acids, a mixed deoxyribonucleic acid solution, T4 DNA polymerases, T4 polynucleotide kinases, and Taq DNA polymerases.

13. The method according to claim 12, wherein in step (A2), the end repair-A-tailing reaction solution is mixed with the product of step (A1) in volume ratio of 1: 4.

14. The method according to claim 2, wherein in step (B1), the fragmentation, the end repair, and the A-tailing reaction are performed in one step by mixing and reacting a fragmentation-end repair-A-tailing reaction solution with the nucleic acid sample, to obtain the product of step (B1); and
the fragmentation-end repair-A-tailing reaction solution contains fragmentmase, a fragmentmase reaction buffer, adenylate deoxyribonucleic acids, a mixed deoxyribonucleic acid solution, T4 DNA polymerases, Taq DNA polymerases, and a TE buffer.

15. The method according to claim 3, wherein in step (C1), the fragmentation and the end repair are performed in one step by mixing and reacting a fragmentation-end repair reaction solution with the nucleic acid sample, to obtain the product of step (C1); and
the fragmentation-end repair reaction solution contains fragmentmase, a fragmentmase reaction buffer, a mixed deoxyribonucleic acid solution, DNA polymerase I, and MgCl₂.

16. The method according to any one of claims 1 to 25, wherein:
in step (A3), (B2), or (C3), the adapter is formed by annealing a B strand and a T strand;
a 3'-end of the B strand is complementary with a 5'-end of the T strand, and the remaining region of the B strand is non-complementary and unpaired with the remaining region of the T stand;
the 3'-end of the B strand has a protruding dT;
the non-complementary region of the B strand and/or the non-complementary region of the T strand contain a barcode for identifying different samples; and
a 5'-end of the B strand and the 5'-end of the T strand are each modified with a phosphate group or ligated with a single-stranded oligonucleotide fragment having a U-base at 3'-end.

17. The method according to claim 16, wherein the adapter is any one of:
adapter 1, formed by annealing a single-stranded DNA set forth in SEQ ID NO: 1 with a phosphate group-modified 5'-end and a single-stranded DNA set forth in SEQ ID NO: 2 with a phosphate group-modified 5'-end;
adapter 2, formed by annealing a single-stranded DNA set forth in SEQ ID NO: 3 or SEQ ID NO: 4 and a single-stranded DNA set forth in SEQ ID NO: 2;
adapter 3, formed by annealing a single-stranded DNA set forth in SEQ ID NO: 5 and a single-stranded DNA set forth in SEQ ID NO: 6; and
adapter 4, formed by annealing a single-stranded DNA set forth in SEQ ID NO: 7 and a single-stranded DNA set forth in SEQ ID NO: 6.

18. The method according to any one of claims 1 to 17, wherein:
in step (A3), (B2), or (C3), the adapter is ligated to the product of step (A2), (B1), or (C2) by mixing and reacting the adapter and the product of step (A2), (B1), or (C2) with a ligation reaction solution, to obtain the product of step (A3), (B2), or (C3); and
the ligation reaction solution contains a T4 polynucleotide kinase buffer, adenylate ribonucleic acids, PEG8000, T4 DNA ligases, and enzyme-free water.

19. The method according to claim 18, wherein:
in step (A3), (B2), or (C3), the adapter, the product of step (A2), (B1), or (C2), and the ligation reaction solution are mixed by mixing an adapter solution containing the adapter and the product of step (A2), (B1), or (C2) with the ligation reaction solution in a volume ratio of (1 to 5): 50: (25 to 29); and
a concentration of the adapter in the adapter solution is 6 µM or 1 µM.

20. The method according to claim 18 or 19, wherein:
in step (A3), (B2), or (C3), the adapter, the product of step (A2), (B1), or (C2), and the ligation reaction solution, after being mixed, react at 25°C for 10 min to 30 min and are kept at 4°C.

21. The method according to any one of claims 1 to 20, wherein in step (A4), (B3), or (C4), the product of step (A3), (B2), or (C3) is purified before the single-strand cyclization and the rolling circle replication.

22. The method according to any one of claims 1 to 21, wherein in step (A4), (B3), or (C4), said obtaining DNA nanoballs by performing the single-strand cyclization and the rolling circle replication on the product of step (A3), (B2), or (C3) comprises any one of:
(a1) sequentially performing single-strand cyclization, linear single strand digestion, purification, and rolling circle replication, to obtain the DNA nanoballs; and
(a2) performing the single-strand cyclization and the rolling circle replication in one step, to obtain the DNA nanoballs.

23. The method according to claim 22, wherein in step (a1), the single-strand cyclization is performed in a manner I or a manner II;
wherein the manner I comprises the following steps:
I-1: incubating the product of step (A3), (B2), or (C3) at 95°C for 3 min and at 4°C for 10 min to obtain an incubation product; and
I-2: mixing and reacting a single-strand cyclization reaction solution 1 with the incubation product of step I-1 to obtain a cyclization product, wherein the single-strand cyclization reaction solution 1 contains a TA buffer, adenylate ribonucleic acids, mediation fragments, T4 DNA ligases, and enzyme-free water, and wherein the mediation fragments are each a single-stranded DNA having a 5'-end reversely complementary to a'-end of the B strand constituting the adapter, and a 3'-end reversely complementary to a 3'-end of the T strand constituting the adapter;
wherein the manner II comprises the following steps:
mixing the product of step (A3), (B2), or (C3) with the mediation fragments and a NaOH solution;
placing the mixture at the room temperature for 5 min;
adding a Tris-HCl solution to the mixture and mixing; and
adding a single-strand cyclization reaction solution 2 for reaction to obtain a cyclization product, the single-strand cyclization reaction solution 2 containing a TA buffer, adenylate ribonucleic acids, and T4 DNA ligases.

24. The method according to claim 23, wherein when the adapter is the adapter 1 or the adapter 2 according to claim 17, the mediation fragments have each a nucleotide sequence set forth in SEQ ID NO: 8.

25. The method according to any one of claims 22 to 24, wherein in step (a1), the linear single strand digestion comprises the following steps:
mixing and reacting a digestion reaction solution with the cyclization product to obtain a digestion product, the digestion reaction solution containing a TA buffer, ExoI enzymes, ExoIII enzymes, and enzyme-free water.

26. A method for constructing a DNA library applicable to PCR-free high-throughput sequencing, comprising steps (A1) to (A4), steps (B1) to (B3), or steps (C1) to (C4) according to any one of claims 1 to 32.

27. A DNA library constructed by the method according to claim 26.

28. An adapter, being the adapter according to claim 16 or 17.

29. A kit, comprising:
the adapter according to claim 28; and
all or some of:
(b1) the end repair-A-tailing reaction solution according to claim 12 or 13;
(b2) the fragmentation-end repair-A-tailing reaction solution according to claim 14;
(b3) the fragmentation-end repair reaction solution according to claim 15;
(b4) the ligation reaction solution according to claim 18;
(b5) the single-strand cyclization reaction solution 1 or the single-strand cyclization reaction solution 2 according to claim 23; and
(b6) the digestion reaction solution according to claim 25.

30. A system, comprising:
the kit according to claim 29; and
a DNBSEQ sequencing reagent and/or device.

31. Use of the DNA library according to claim 27, the adapter according to claim 28, the kit according to claim 29 or the system according to claim 30 in PCR-free high-throughput sequencing.

32. Use of the adapter according to claim 28 or the kit according to claim 29 in construction of the DNA library according to claim 27.
